# EUROPÄISCHE PATENTSCHRIFT

(11) **EP 4 322 875 B1**
(45) Veröffentlichungstag und Bekanntmachung des Hinweises auf die Patenterteilung: **02.04.2025**
(21) Anmeldenummer: 22723556.1
(22) Anmeldetag: 13.04.2022
(51) Int. Cl.: A61B 17/70, A61B 17/86

(54) **OSTEOSYNTHESEVORRICHTUNG ZUR WIRBELSÄULENBEHANDLUNG UND BEFESTIGUNGSSYSTEM FÜR DIESE**
OSTEOSYNTHESIS DEVICE FOR SPINAL TREATMENT, AND ATTACHMENT SYSTEM FOR THIS
DISPOSITIF D'OSTÉOSYNTHÈSE POUR LE TRAITEMENT DE LA COLONNE VERTÉBRALE ET SYSTÈME DE FIXATION ASSOCIÉ

(30) Priorität: 13.04.2021 DE 102021001896; 12.04.2022 DE 102022108980
(43) Veröffentlichungstag der Anmeldung: 21.02.2024
(73) Patentinhaber: Ortho Hub Ventures UG (Haftungsbeschränkt), 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2022/059942
(87) Internationale Veröffentlichungsnummer: WO 2022/219077

(56) Entgegenhaltungen:
- EP-A1- 3 128 934
- WO-A1-2018/024414
- DE-B3- 102018 102 173
- US-A1- 2012 245 640

## Beschreibung

### Gebiet der Erfindung

Die Erfindung betrifft ein Befestigungssystem zur Wirbelsäulenbehandlung und ein Befestigungssystem für diese.

### Stand der Technik

Im Stand der Technik sind verschiedene Osteosynthesevorrichtungen zur Versorgung der Wirbelsäule, wie beispielsweise Pedikelschrauben, bekannt. Solche Osteosynthesevorrichtungen werden dafür verwendet, Wirbelsäulenfehlstellungen zu korrigieren oder Frakturen zu stabilisieren, indem die Osteosynthesevorrichtungen in den Wirbelknochen eingebracht und befestigt werden und dann über Längsstäbe, oder sogenannte Verbindungsstäbe, miteinander verbunden werden, um die Wirbel in einer gewünschten Stellung miteinander zu fixieren. Dabei werden die Längsstäbe mit Hilfe von Verriegelungs-Elementen, wie beispielsweise Madenschrauben oder sonstigen Verschluss-Elementen, an den Osteosynthesevorrichtungen montiert und rutschfest fixiert. Als Osteosynthesevorrichtungen kommen vorzugsweise Pedikelschrauben zum Einsatz, die einen Knochenanker besitzen, welcher in wenigstens einer Ebene mit einem Gabelkopf verschwenkbar gelagert ist und bei fixierter Madenschraube winkelstabil ist. Als Knochenanker kommen vorzugsweise Knochenschrauben mit einem Kugelkopf zum Einsatz. Regulär werden Osteosynthesevorrichtungen mit Knochenanker und Gabelkopf so montiert, dass der Knochenanker von proximal kommend in den Gabelkopf durch die distale Öffnung des Gabelkopfes geführt wird. Das funktioniert nur, wenn der äußere Durchmesser des Knochenankerschafts kleiner ist als der Kugelkopfdurchmesser des Knochenankers und der äußere Durchmesser des Knochenankerschafts kleiner ist als der Durchmesser der distalen Öffnung des Gabelkopfes. Problematisch ist die Montage, wenn der äußere Durchmesser des Knochenankerschafts größer ist als der Öffnungsdurchmessers der Gabelkopfes und/oder des Kugelkopfdurchmesser des Knochenankers.

Aus dem Stand der Technik ist eine Pedikelschraube (DE102011053295A1) bekannt, welche temporär verriegelbar ist und somit ein erweitertes Anwendungsspektrum bei der Versorgung von Wirbelsäuleninstabilitäten erlaubt. Damit ist es möglich, polyaxial bewegliche Pedikelschrauben in einer beliebigen Orientierung in den Wirbelknochen einzubringen und im Anschluss kann der Anwender den Winkel zwischen Gabelkopf und Knochenanker temporär verklemmen. Mit einer temporär gesperrten Polyaxialität ist es möglich, dass über den Gabelkopf Korrekturkräfte auf den Knochenanker eingeleitet werden und diese sich direkt auf die Stellung und Position der Wirbelknochen auswirkt. Ohne diese temporäre Verklemmung sind solche Korrekturmanöver nicht oder nur schwer ausführbar. Bei einer solchen Anordnung ist es erforderlich, dass das Druckstück mit Hilfe eines Instruments permanent geklemmt wird und das Instrument jederzeit an der Schraube befestigt ist, damit die für die temporäre Klemmung notwendige Kompressionskraft aufrechterhalten wird. Bei manchen Wirbelsäulenkorrekturmanövern, wie beispielsweise bei der Korrektur von Deformitäten und Skoliosen, ist kein Platz für derartige Instrumente. Deshalb wäre es wünschenswert, ein Schraubenimplantat vorzusehen, welches eigenständig in der Lage ist, die temporäre Klemmung aufrechtzuerhalten, sobald die Instrumente entfernt sind.

Aus der Anmeldung DE102018102173B3 ist ein Pedikelschraubenanker bekannt, bei dem die temporäre Kompressionskraft permanent aufrechterhalten bleibt, indem ein lösbares Stift-Element vorgesehen ist. Allerdings erfordert dieser Aufbau für die Erzeugung der temporären Klemmung eine hebelartige Betätigung des Druckstücks. Daraus resultiert eine erhöhte kombinierte Kompressions- und Biegebeanspruchung des Druckstücks, was zu einer mechanischen Limitation der maximalen temporären Klemmwirkung führt. Des Weiteren ist es notwendig, dass das Druckstück in seiner Materialdicke so dimensioniert ist, dass diese Belastungen sich nicht zerstörend auswirken. Dies hat zur Folge, dass eine solche Pedikelschraube in ihrer Bauhöhe größer ist als eine reguläre Pedikelschraube. Deshalb ist es wünschenswert, dass die temporäre Klemmwirkung nicht über das Druckstück selbst eingeleitet wird, sondern direkt am Knochenanker-Kopfbereich angreift. Damit ist das Druckstück bei der temporären Klemmung mechanisch entkoppelt und die Osteosynthesevorrichtung kann Reserven bzgl. in der maximalen Klemmwirkung bereitstellen.

Des Weiteren ist zu erkennen, dass keine dieser temporär klemmbaren Pedikelschrauben-Konzepte in der Lage ist, Schraubenschäfte von distal kommend aufzunehmen. Mit ihnen können nur Knochenanker angewendet werden, bei denen der äußere Durchmesser des Knochenankerschafts kleiner ist als der Durchmesser der distalen Öffnung des Gabelkopfes.

Aus dem Stand der Technik bekannte Osteosynthesevorrichtungen beziehungsweise Pedikelschrauben sind dementsprechend unflexibel und unsicher hinsichtlich Positionierung sowie Fixierung und benötigen viel Bauraum bei der Implantation und Einstellung, aber auch hinsichtlich des dauerhaften Verbleibs im Patienten. Darüber hinaus weisen sie eine schlechte Handhabbarkeit insbesondere für den Operateur auf.

### Darstellung der Erfindung

Aufgabe der vorliegenden Erfindung ist es daher, zu ermöglichen, dass Osteosynthesevorrichtungen einfach, flexibel und sicher handhabbar, insbesondere einfach, flexibel und sicher positionierbar und fixierbar, sind und einen geringen Bauraum aufweisen.

Die Aufgabe wird gelöst durch ein Befestigungssystem nach Anspruch 1. Weitere die Erfindung ausgestaltende Merkmale sind in den abhängigen Ansprüchen enthalten.

So wird es ermöglicht, eine temporär klemmbare Osteosynthesevorrichtung, insbesondere Pedikelschraube, bereitzustellen, die es erlaubt, dass ein Knochenanker von distal kommend montierbar ist und dass das Druckstück bei Betätigung der temporären Klemmung nicht belastet, sondern erst bei finaler Verriegelung der Osteosynthesevorrichtung mit den Verbindungsstab und dem Verschluss-Element mit einer Druckkraft beaufschlagt wird. Dadurch ergibt sich eine Modularität des Schraubensystems, was Vorteile bzgl. der reduzierten Kapitalbindung beim Anwender hat und es kann ein insgesamt größeres Schrauben-Portfolio angeboten werden. Des Weiteren ergibt sich durch die Kraft-Fluss-Aufteilung bei der temporären Klemmung ohne die mechanische Beteiligung des Druckstücks ein deutlicher Stabilitätsgewinn gegenüber bisherigen Konzepten und die Pedikelschraube kann in ihrer Bauhöhe kleiner gestaltet werden. Gleichzeitig soll das erfindungsgemäße Konzept die temporär erzeugte Klemmung selbstständig aufrechterhalten, auch wenn sämtliche Instrumente entfernt sind.

Im Folgenden werden Komponenten, Befestigungssysteme und Osteosynthesevorrichtungen dargestellt, mittels derer ein Knochenanker in seiner Ausrichtung beziehungsweise Lage fixiert werden kann. Dabei wird erfindungsgemäß - wo dies für das Verständnis hilfreich erscheint - zwischen Vorfixieren und finalem Fixieren unterschieden. Als Vorfixieren beziehungsweise vorfixiert wird erfindungsgemäß das Fixieren des Knochenankers in seiner Ausrichtung beziehungsweise Lage verstanden, mit dem erreicht wird, dass während der operativen Implantation eines Befestigungssystems beziehungsweise einer Osteosynthesevorrichtung eine temporäre Lage- beziehungsweise Ausrichtungssicherung des Knochenankers gewährleistet werden kann, beispielsweise um die Osteosynthesevorrichtung mit weiteren Komponenten, wie weiteren Osteosynthesevorrichtungen, zu verbinden beziehungsweise diese präzise einzulegen und/oder zu positionieren. Demgegenüber wird als finales Fixieren beziehungsweise final fixiert erfindungsgemäß das Fixieren des Knochenankers verstanden, mit dem erreicht wird, dass dieser einschließlich gegebenenfalls des beziehungsweise der an beziehungsweise mit diesem fixierten Befestigungssystems beziehungsweise Osteosynthesevorrichtung insbesondere auch gegenüber bewegungsinduzierter Belastungen lagestabil im Patienten verbleibt.

Weiterhin wird im Folgenden die räumliche Angabe proximal verwendet, womit bei der finalen Ausrichtung des Befestigungssystems beziehungsweise der Osteosynthesevorrichtung eine Nähe zum Operateur gemeint ist. Für demgegenüber befindliche Komponenten wird die räumliche Angabe distal verwendet, womit bei der finalen Ausrichtung des Befestigungssystems beziehungsweise der Osteosynthesevorrichtung ein Abstand zum Operateur gemeint ist beziehungsweise vom proximalen Ende betrachtet das gegenüberliegende Ende des Befestigungssystems beziehungsweise der Osteosynthesevorrichtung.

Ein erfindungsgemäßes Befestigungssystem für eine Osteosynthesevorrichtung, insbesondere zur Wirbelsäulenbehandlung, weist einen Hauptkörper mit einem ersten Aufnahmebereich, der zur Aufnahme eines Verbindungselementes ausgebildet ist, einem zweiten Aufnahmebereich, der zur Aufnahme eines Knochenankers ausgebildet ist, einem dritten Aufnahmebereich aufweisend eine lineare Quer-Öffnung, wobei die Quer-Öffnung an ihrem ersten Ende eine erste Öffnung und an ihrem zweiten Ende eine zweite Öffnung aufweist, und einer Axial-Öffnung zur Aufnahme und Führung eines Stell-Elementes auf. Ferner weist das erfindungsgemäße Befestigungssystem ein Fixier-Element aufweisend einen ersten Fixierstift, und einen Lastaufnahmebereich, wobei der erste Fixierstift über die erste Öffnung in die Quer-Öffnung einführbar ist und das Fixier-Element länger als die Quer-Öffnung ist, und wobei der erste Fixierstift an seinem dem Lastaufnahmebereich entfernten Ende eine Kontaktfläche aufweist, und ein Stell-Element aufweisend einen Lasteinleitungsbereich, wobei das Stell-Element mittels der Axial-Öffnung derart positionierbar und führbar ist, dass mittels des durch den Lasteinleitungsbereich auf den Lastaufnahmebereich ausgeübten Druckes das Fixier-Element entlang der Quer-Öffnung in Richtung des zweiten Aufnahmebereiches führbar ist, auf.

Die Quer-Öffnung des Druckstücks kann beispielsweise als Ausschnitt ausgebildet sein.

Der Lasteinleitungsbereich wird im Folgenden auch als Kontakt-Bereich bezeichnet. Der Lastaufnahmebereich wird im Folgenden auch als Kontakt-Bereich bezeichnet. Die Kontaktfläche wird im Folgenden auch als Kontakt-Bereich bezeichnet.

Der zweite Aufnahmebereich kann dabei, falls der Knochenanker ein als Kugelkopf ausgebildetes Ende aufweist, als Kugelkopfaufnahmebereich ausgebildet sein. Der zweite Aufnahmebereich ist dabei vorzugsweise derart ausgestaltet, dass das proximale Ende des Knochenankers in diesen aufnehmbar ist. Dieses proximale Ende des Knochenankers wird im Folgenden auch als Kopfbereich des Knochenankers bezeichnet.

Das Verbindungselement dient zur Verbindung mehrerer Osteosynthesevorrichtungen und ist insbesondere ein Verbindungsstab, besonders bevorzugt ein Rundstab.

Der Knochenanker kann beispielsweise als Knochenschraube oder als Knochennagel ausgestaltet sein.

Vorzugsweise ist der zweite Aufnahmebereich ferner zur lockeren Fixierung des Knochenankers entlang seiner Zentralachse ausgebildet. Die Zentralachse entspricht der Längsachse des Knochenankers. Dadurch kann ein ungewolltes Lösen des Knochenankers vom Befestigungssystem, beispielsweise ein Herausfallen des Knochenankers aus dem Befestigungssystem, verhindert werden, wodurch die Handhabbarkeit verbessert wird.

Vorzugsweise weist die Quer-Öffnung entlang ihrer Führungslinie einen konstanten Querschnitt auf. Derart wird eine sichere Führung des Fixier-Elementes in der Quer-Öffnung ermöglicht.

Besteht eine Vorzugsausrichtung des Fixier-Elementes, ist vorzugsweise der Querschnitt des Teils des Fixier-Elementes, der in der Quer-Öffnung geführt wird, und der Querschnitt der Quer-Öffnung jeweils so ausgebildet, dass mittels der beiden Querschnitte das Fixier-Element nur so in die Quer-Öffnung einführbar ist, dass das Fixier-Element in der Vorzugsausrichtung vorliegt. Derart wird eine zeitsparende und sichere Handhabbarkeit ermöglicht.

Vorzugsweise sind der Querschnitt des Teils des Fixier-Elementes, der in der Quer-Öffnung geführt wird, und der Querschnitt der Quer-Öffnung zueinander komplementär ausgebildet. Derart wird eine sichere Führung des Fixier-Elementes mit geringem Spiel ermöglicht.

Vorzugsweise ist die erste Öffnung innenliegend positioniert. Das heißt, dass der Hauptkörper derart ausgestaltet ist, dass das Fixier-Element über den zweiten Aufnahmebereich in die Quer-Öffnung einführbar ist. Dementsprechend ist die erste Öffnung derart positioniert und ausgestaltet, dass auf der einen Seite der zweite Aufnahmebereich liegt und auf der anderen Seite der dritte Aufnahmebereich liegt. Derart kann erreicht werden, dass bei der Ausgestaltung der Axial-Öffnung nicht vorgesehen werden muss, dass das Fixier-Element von außen in die Quer-Öffnung einführbar sein muss. Derart kann ein reduzierter Bauraum vorgesehen werden, wodurch das Befestigungssystem am Patienten mit einem geringen operativen Aufwand verwendbar ist.

Alternativ kann die erste Öffnung auch außenliegend vorgesehen sein. Dadurch wird es ermöglicht, das Fixier-Element auch dann in die Quer-Öffnung einzuführen, wenn in dem zweiten Aufnahmebereich bereits ein Knochenanker aufgenommen ist.

Vorzugsweise ist die Quer-Öffnung derart ausgestaltet und positioniert, dass abhängig von der Geometrie des proximalen Endes des Knochenankers das Fixier-Element lastoptimiert auf das proximale Ende des Fixier-Elementes geführt werden kann, beispielsweise indem vermieden wird, dass das Fixier-Element auf eine Kante des distalen Endes des Knochenankers trifft.

Vorzugsweise ist die Quer-Öffnung derart ausgestaltet und positioniert, dass abhängig von der Geometrie des proximalen Endes des Knochenankers das Fixier-Element derart auf das proximale Ende des Knochenankers geführt werden kann, dass der Knochenanker unabhängig von seiner Ausrichtung in dem zweiten Aufnahmebereich nicht aus dieser gewünschten Ausrichtung ausgelenkt wird. Dies kann beispielsweise durch einen Bezug auf das distale Ende des Knochenankers zentrierte Führung des Fixier-Elementes auf das distale Ende des Knochenankers erreicht werden. Dadurch wird die Flexibilität des Befestigungssystems erhöht und dessen Handhabbarkeit verbessert.

Indem das Fixier-Element länger ist als die Quer-Öffnung, wird erreicht, dass der erste Fixierstift zumindest teilweise aus der Quer-Öffnung austreten und auf den im zweiten Aufnahmebereich aufgenommenen Knochenanker gepresst werden kann. Derart kann der Knochenanker im zweiten Aufnahmebereich fixiert werden.

Dass das Fixier-Element länger ist als die Quer-Öffnung kann dadurch erreicht werden, dass der Fixierstift länger ausgestaltet ist als die Quer-Öffnung. Die Quer-Öffnung kann aber auch derart ausgestaltet sein, dass der Lastaufnahmebereich zumindest teilweise in diese eingeführt wird, sodass der Fixierstift nicht zwingend länger ausgestaltet sein muss als die Quer-Öffnung. Derart kann eine besonders platzsparende Ausgestaltung erreicht werden.

Vorzugsweise ist der dritte Aufnahmebereich derart ausgestaltet, dass die Quer-Öffnung mit einem Versatz zur Zentralachse des in Neutralstellung in den zweiten Aufnahmebereich aufgenommenen Knochenankers vorgesehen ist, wobei der Versatz mindestens 20°, vorzugsweise 30° und besonders bevorzugt 40° sowie maximal 80°, vorzugsweise 70° und besonders bevorzugt 60° beträgt. Der Versatz bezieht sich dabei darauf, dass das Fixier-Element nicht von proximal nach distal, das heißt entlang der Zentralachse des in Neutralstellung in den zweiten Aufnahmebereich aufgenommenen Knochenankers auf den Knochenanker gepresst wird, sondern von dieser Orientierung beziehungsweise Achse versetzt. Vorzugsweise erfolgt der Versatz dann durch Rotation der Längsachse der Quer-Öffnung um einen Rotationspunkt, der im Wesentlichen zentral im distalen Ende der Knochenschraube positioniert ist. Dadurch wird erreicht, dass der Knochenanker beziehungsweise dessen distales Ende an den Rand des zweiten Aufnahmebereichs gepresst wird. Derart wird eine verstärkte Fixierwirkung erreicht. Die Neutralstellung des Knochenankers entspricht dabei der Stellung von Knochenanker und Hauptkörper zueinander, bei der die Zentralachse des Knochenankers im Wesentlichen entlang der Richtung des Hauptkörpers verläuft, die sich von proximal beim ersten Aufnahmebereich beginnend zum zweiten Aufnahmebereich hin, also nach distal gerichtet, ergibt.

Vorzugsweise ist das distale Ende des Knochenankers kugelartig ausgestaltet, beispielsweise als Kugelkopf, und der zweite Aufnahmebereich komplementär dazu ebenfalls kugelartig. Dadurch kann sichergestellt werden, dass schon bei geringen Versatzwinkeln das distale Ende des Knochenankers zumindest teilweise senkrecht auf zumindest einen Flächenanteil des zweiten Aufnahmebereichs gepresst wird.

Vorzugsweise sind der Lasteinleitungsbereich und der Lastaufnahmebereich zueinander komplementär ausgebildet. Derart kann erreicht werden, dass die durch das Stell-Element auf das Fixier-Element zu übertragende Last über eine möglichst große Kontaktfläche übertragen wird, wodurch der dabei auf die Kontaktfläche ausgeübte Druck minimiert wird. Dadurch wird eine sichere und robuste Führung des Fixier-Elementes ermöglicht.

Sofern das Stell-Element ein Gewinde aufweist, also beispielsweise als Belastungsschraube ausgeführt ist und der Lasteinleitungsbereich am distalen Ende des Stell-Elementes vorgesehen ist, kann der Lastaufnahmebereich konkav vorgesehen sein. Derart kann bei Rotation des Stell-Elementes die Last sicher übertragen werden.

Vorzugsweise sind die Kontaktflächen des Lasteinleitungsbereiches und des Lastaufnahmebereiches im Wesentlichen senkrecht zur Längsachse der Quer-Öffnung orientiert. Dadurch kann erreicht werden, dass die Last entlang der Quer-Öffnung auf das Fixier-Element ausgeübt wird, wodurch das Verkant- beziehungsweise Verkeilrisiko des Fixier-Elementes minimiert wird. Dadurch wird eine sichere Handhabung des Befestigungssystems beziehungsweise der Osteosynthesevorrichtung ermöglicht.

Vorzugsweise weist das Befestigungssystem ferner ein Druckstück auf, wobei das Druckstück einen zu dem ersten Aufnahmebereich des Befestigungssystems äquivalenten ersten Aufnahmebereich, einen zu dem zweiten Aufnahmebereich des Befestigungssystems äquivalenten und mehrere Federelemente aufweisenden zweiten Aufnahmebereich sowie einen zu dem dritten Aufnahmebereich des Hauptkörpers äquivalenten und eine lineare Quer-Öffnung aufweisenden dritten Aufnahmebereich aufweist, und wobei das Druckstück über den ersten Aufnahmebereich des Hauptkörpers in den Hauptkörper einsetzbar ist. Äquivalent bedeutet hierbei, dass im eingesetzten Zustand des Druckstückes das Verbindungselement über die beiden ersten Aufnahmebereiche, das heißt über den ersten Aufnahmebereich des Hauptkörpers und den ersten Aufnahmebereich des Druckstücks, in das Befestigungssystem aufgenommen werden kann, der Knochenanker über die beiden zweiten Aufnahmebereiche, das heißt den zweiten Aufnahmebereich des Hauptkörpers und den zweiten Aufnahmebereich des Druckstücks, entlang dessen Zentralachse locker fixiert werden kann, und das Fixier-Element über die ersten Öffnungen der beiden dritten Aufnahmebereiche, das heißt über die erste Öffnung des Hauptkörpers und über die erste Öffnung des Druckstücks, in deren Quer-Öffnungen einführbar ist. Das Druckstück dient dann dazu, eine sichere finale Fixierung des Knochenankers zu ermöglichen und dann eine Stabilisierung des geschädigten Körperbereiches gewährleistet wird. Diese finale Fixierung wird dadurch erreicht, dass das Verbindungselement derart auf beziehungsweise in den ersten Aufnahmebereich des Druckstücks gepresst wird, dass der zweite Aufnahmebereich des Druckstücks derart verformt wird, dass die Federelemente aufeinander zubewegt werden. Befindet sich nun ein Knochenanker beziehungsweise das proximale Ende eines Knochenankers in dem zweiten Aufnahmebereich des Druckstücks, werden die Federelemente auf diesen beziehungsweise dieses gepresst, wodurch ein Reibschluss beziehungsweise Kraftschluss zwischen dem zweiten Aufnahmebereich des Druckstücks und dem Knochenanker beziehungsweise dessen proximalem Ende resultiert. Der erste Aufnahmebereich des Druckstücks wird im Folgenden auch als Stablager bezeichnet.

Der zweite Aufnahmebereich des Druckstücks wird im Folgenden auch als Knochenanker-Kopfbereich bezeichnet. Das Federelement wird im Folgenden auch als Arm beziehungsweise federelastischer Arm bezeichnet.

Es ist ebenfalls möglich, dass derartige Federelemente im zweiten Aufnahmebereich des Hauptkörpers vorgesehen sind, mittels derer dann ein solcher Reibschluss beziehungsweise Kraftschluss erreicht wird.

Vorzugsweise ist der dritte Aufnahmebereich des Druckstücks derart ausgestaltet, dass zwischen dem Fixier-Element und dem Druckstück keine kraftschlüssige Verbindung resultieren kann, besonders vorzugsweise dies sogar im Zustand des auf den Knochenanker gepressten Fixier-Elementes. Derart wird eine sichere Vorfixierung des Knochenankers ermöglicht, wobei gleichzeitig ein Verklemmen beziehungsweise Verkanten des Druckstücks vermieden wird, sodass letztlich ebenfalls eine sichere finale Fixierung des Knochenankers ermöglicht wird. Um dies zu erreichen, können beispielsweise großflächige Aussparungen in der Quer-Öffnung des Druckstücks vorgesehen sein, die vorzugsweise komplementär zu dem ersten Fixierstift beziehungsweise gegebenenfalls komplementär zu weiteren Fixierstiften ausgestaltet sind.

Vorzugsweise ist das Druckstück ferner derart ausgestaltet, dass eine erste Einführstellung ermöglicht wird, in der der Knochenanker in den zweiten Aufnahmebereich des Hauptkörpers einführbar ist und eine zweite Einführstellung ermöglicht wird, mit der der Knochenanker nicht mehr aus dem Hauptkörper entfernbar ist. Besonders vorzugsweise ist dabei von proximal nach distal einstellbar, dass das Druckstück in den ersten Aufnahmebereich des Hauptkörpers eingeführt wird, wobei das Druckstück nicht bis zum distalen Ende des ersten Aufnahmebereichs des Hauptkörpers versetzt wird. Davon ausgehend ist dann die zweite Einführstellung erreichbar, indem das Druckstück weiter nach distal in den ersten Aufnahmebereich des Hauptkörpers eingesetzt beziehungsweise eingeschoben wird. Während des Verschiebens des Druckstückes von der ersten Einführstellung zur zweiten Einführstellung können die Federelemente des Druckstücks kurzzeitig radial ausgelenkt werden, um anschließend wieder in deren Neutralstellung zurückzuschwenken. Wurde das Druckstück in die zweite Einführstellung versetzt, versperren die distalen Enden der Federelemente das distale Ende des zweiten Aufnahmebereiches des Hauptkörpers, sodass der Knochenanker nicht mehr nach distal aus dem Hauptkörper entfernbar ist. Derart wird eine Verliersicherung für den Knochenanker ermöglicht, wodurch eine sichere Handhabbarkeit des Befestigungssystems beziehungsweise der Osteosynthesevorrichtung ermöglicht wird. Dafür kann der zweite Aufnahmebereich nach distal verjüngend ausgebildet sein.

Vorzugsweise weist die Axial-Öffnung ein Innengewinde auf und weist das Stell-Element ein Außengewinde auf, und ist das Innengewinde der Axial-Öffnung komplementär zu dem Außengewinde des Stell-Elementes ausgebildet. Derart wird es ermöglicht, eine Last auf das Fixier-Element mittels Anschrauben des Stell-Elementes aufzubringen. Dadurch wird ein präzises und sicheres Fixieren des Knochenankers ermöglicht. Das Außengewinde des Stell-Elementes wird im Folgenden auch als Gewinde bezeichnet.

Vorzugsweise weist der erste Aufnahmebereich des Hauptkörpers proximal ferner ein Innengewinde auf. Derart wird es ermöglicht, dass das Verbindungselement mittels einer Schraube, beispielsweise mittels einer Madenschraube, in den ersten Aufnahmebereich beziehungsweise in die ersten Aufnahmebereiche hineingepresst werden kann.

Vorzugsweise ist der Gewindedurchmesser der Axial-Öffnung und der Gewindedurchmesser des Stell-Elementes geringer als der Gewindedurchmesser des ersten Aufnahmebereiches des Hauptkörpers. Derart kann ein geringer Platzbedarf des Befestigungssystems erreicht werden, wodurch die während der operativen Implantation in den Patienten auftretende Gewebeschädigung minimiert wird.

Vorzugsweise weisen das Innengewinde der Axial-Öffnung und das Außengewinde des Stell-Elementes eine Gewindesteigung auf, die geringer ist als die Gewindesteigung des Innengewindes des ersten Aufnahmebereiches des Hauptkörpers. Derart kann das zum Belasten des Stell-Elementes anzuwendende Anzugsmoment reduziert werden, wodurch die Torsionsbelastung des Stell-Elementes reduziert wird und gleichzeitig ein sicheres Fixieren des Knochenankers durch das Fixier-Element ermöglicht wird. Dies ist vor allem von Vorteil, wenn das Stell-Element mit geringerer Dimensionierung, beispielsweise mit geringerem Umfang ausgeführt ist, als die in den ersten Aufnahmebereich des Hauptkörpers aufzunehmende Madenschraube, da dann das Stell-Element eine geringere Torsionsfestigkeit aufweist.

Vorzugsweise weist das Innengewinde der Axial-Öffnung und das Außengewinde des Stell-Elementes eine Steigung von maximal 1,00 mm pro Umdrehung, vorzugsweise von weniger als 0,85 mm pro Umdrehung, besonders vorzugsweise von weniger als 0,70 mm pro Umdrehung und von minimal 0,1 mm pro Umdrehung, vorzugsweise 0,2 mm pro Umdrehung, besonders vorzugsweise 0,3 mm pro Umdrehung auf. Derart kann erreicht werden, dass einerseits die Torsionsbelastung des Stell-Elementes reduziert wird und gleichzeitig das Stell-Element in der Axial-Öffnung eine deutlich höhere axiale Vorspannung erzeugt, wodurch die Handhabbarkeit des Befestigungssystems verbessert wird.

Vorzugsweise verläuft das Innengewinde der Axial-Öffnung im Wesentlichen parallel zu der Zentralachse des in Neutralstellung in den zweiten Aufnahmebereich aufgenommenen Knochenankers. Derart wird eine platzsparende Bauweise des Befestigungselementes ermöglicht, wodurch die während der operativen Implantation der Osteosynthesevorrichtung auftretende Gewebeschädigung reduziert wird. Ferner wird es dadurch ermöglicht, das Stell-Element und die in den ersten Aufnahmebereich des Hauptkörpers aufgenommene Madenschraube in ähnlicher Weise und nahe beieinander liegend zu betätigen, wodurch die Handhabbarkeit verbessert wird.

Es ist ebenfalls möglich, dass die Axial-Öffnung parallel zur Hauptachse der Quer-Öffnung des Hauptkörpers ausgerichtet ist. Derart kann die Kraftschlusswirkung zwischen Stell-Element und Fixier-Element sowie letztlich zwischen Fixier-Element und Knochenanker maximiert werden, wodurch wiederum die Zuverlässigkeit und Stabilität des Befestigungssystems erhöht wird.

Es ist ferner möglich, dass die Quer-Öffnung des Hauptkörpers im Wesentlichen senkrecht zur Zentralachse des in Neutralstellung in den zweiten Aufnahmebereich des Hauptkörpers aufgenommenen Knochenankers ausgerichtet ist. Derart kann eine maximale Klemmwirkung bezüglich des Knochenankers erreicht werden.

Vorzugsweise weist die Axial-Öffnung zumindest eine Verdrücköffnung auf, wobei die Verdrücköffnung am distalen Ende des Innengewindes positioniert ist, und wobei die Verdrücköffnung derart ausgebildet ist, dass über diese ein Verdrücken des Außengewindes des Stell-Elementes ermöglicht wird. Derart kann im Sinne einer Verliersicherung verhindert werden, dass das Stell-Element beispielsweise selbsttätig aus der Axial-Öffnung herausgelöst wird, wodurch die Handhabbarkeit des Befestigungssystems verbessert wird. Als Verdrücken wird dabei eine lokale Verformung des Außengewindes des Stell-Elementes verstanden, die derart intensiv ist, dass bei Eingriff des verformten Außengewindeabschnittes in ein zu diesem Außengewinde grundsätzlich komplementäres Innengewinde, der verformte beziehungsweise verdrückte Außengewindeabschnitt im Innengewinde verklemmt.

Ebenfalls möglich ist ein Verdrücken des Außengewindes über die Außenwand der Axial-Öffnung. Dabei bestünde allerdings das Risiko, dass die Axial-Öffnung mitverformt wird, wodurch das Stell-Element möglicherweise vollständig festgeklemmt würde, das heißt weder aus der Axial-Öffnung herausgedreht, noch in diese hereingedreht werden könnte. Daher ist es vorteilhaft, den Hauptkörper derart auszugestalten, dass ein Verdrücken des Außengewindes des Stell-Elementes ohne Schädigung des Innengewindes der Axial-Öffnung und außerdem in Bezug auf das Stell-Element lokal beschränkt und dieses nicht substantiell schädigend ausführbar ist.

Vorzugsweise weist die Axial-Öffnung mehrere Verdrücköffnungen auf, beispielsweise zwei, die besonders bevorzugt einander gegenüberliegend positioniert sind. Derart wird ein gleichmäßiges Verdrücken ermöglicht, ohne gegebenenfalls die umgebenden Strukturen schädigend zu belasten.

Vorzugsweise endet das in der Axial-Öffnung ausgebildete Innengewinde nach distal hin vor der Verdrücköffnung beziehungsweise den Verdrücköffnungen. Derart ist es auch nach erfolgter Verdrückung des Außengewindes des Stell-Elementes möglich, dieses nach distal hin weiter in die Axial-Öffnung einzuschrauben, während gleichzeitig ein Ausschrauben nach proximal hin verhindert wird.

Vorzugsweise weist das Fixier-Element ferner einen zweiten Fixierstift auf. Derart wird es ermöglicht, dass über das Fixier-Element der Knochenanker über zwei Belastungspunkte geklemmt wird. Die Belastungspunkte ergeben sich dabei über die Kontaktflächen zwischen den distalen Enden des ersten und zweiten Fixierstiftes und dem Knochenanker. Ausgehend von der jeweiligen Kontaktfläche, dem sich auf der gegenüberliegenden Seite des Knochenankers befindenden Bereich und der Lasteinleitungsrichtung ergibt sich jeweils eine Belastungsrichtung beziehungsweise Belastungsachse. Wird nun der fixierte Knochenanker belastet, beispielsweise während der operativen Implantation, resultieren teilweise unterschiedliche Klemmwiderstände. Erfolgt diese Belastung entlang der Belastungsachse, resultiert ein großer Klemmwiderstand. Erfolgt die Belastung jedoch abseits der Belastungsachse, beispielsweise in Form einer Torsionsbelastung um die Belastungsachse herum, resultiert ein geringer Klemmwiderstand. Das kann dazu führen, dass die Klemmwirkung nicht hinsichtlich jeder möglichen Belastung im erforderlichen Umfang erbracht werden kann. Es kann ebenfalls dazu führen, dass um hinsichtlich jeder möglichen Belastung die erforderliche Klemmwirkung erbringen zu können, das Stell-Element verstärkt betätigt wird, wodurch dann das Fixier-Element verstärkt auf den Knochenanker gepresst wird. Dadurch steigt allerdings die Gefahr eines Materialversagens. Werden nun mehrere Belastungspunkte ermöglicht, kann diese Gefahr wiederum minimiert werden und gleichzeitig hinsichtlich jeder möglichen Belastungssituation eine sichere Klemmwirkung erreicht werden.

Es ist ebenfalls möglich, einen einzigen Fixierstift derart auszugestalten, dass dieser eine Kontaktfläche aufweist, die im Eingriff mit dem Knochenanker dazu führt, dass mehrere Belastungsachsen resultieren. Das kann beispielsweise dadurch erreicht werden, dass auf der Kontaktfläche zwei zueinander beabstandete Erhebungen vorgesehen sind.

Vorzugsweise ist das Fixier-Element derart ausgestaltet, dass bei Klemmung des Knochenankers durch dieses eine Dreipunktlagerung beziehungsweise Dreipunktbelastung erreicht wird. Derart wird auf einfache Weise hinsichtlich jeder Belastungssituation des Knochenankers dessen sichere Klemmung ermöglicht.

Vorzugsweise ist die Kontaktfläche des ersten Fixierstiftes oder gegebenenfalls des ersten und/oder zweiten Fixierstiftes komplementär zu einer entsprechenden Kontaktfläche des Knochenankers ausgebildet. Derart wird eine sichere und materialschonende Lastübertragung von den Fixierstiften auf den Knochenanker ermöglicht.

Besonders vorzugsweise ist die Kontaktfläche des ersten Fixierstiftes oder gegebenenfalls des ersten und/oder zweiten Fixierstiftes derart ausgestaltet, dass der Kontaktbereich zwischen dem ersten Fixierstift oder gegebenenfalls dem ersten und/oder zweiten Fixierstift sowie dem Knochenanker maximiert wird. Derart wird auch die entsprechende Reibfläche maximiert, wodurch wiederum die Kraftschlusswirkung maximiert wird.

Vorzugsweise ist das Befestigungssystem für einen Knochenanker mit Kugelkopf vorgesehen. Derart kann mit einer an diesen Kugelkopf angepassten sphärisch-konkav gekrümmten Kontaktfläche des Fixierstiftes ermöglicht werden, dass der Knochenanker unabhängig von seiner Verdrehposition sicher vorfixiert werden kann. Als Verdrehposition wird jede sich geometrisch anhand des Hauptkörpers und des proximalen Endes des Knochenankers ergebende mögliche Position des Knochenankers verstanden.

Vorzugsweise ist der approximierte Durchmesser der sphärisch-konkav gekrümmten Kontaktfläche des ersten Fixierstiftes oder gegebenenfalls des ersten und/oder zweiten Fixierstiftes geringer als der Durchmesser des als Kugelkopf ausgebildeten proximalen Endes des Knochenankers. Derart wird es ermöglicht, dass bei Aufpressen des ersten Fixierstiftes oder gegebenenfalls des ersten und/oder zweiten Fixierstiftes auf das proximale Ende des Knochenankers, die sich ergebende Kontaktfläche zusätzlich scherbelastet wird, beispielsweise in Form einer Umkrimpung. Ferner wird es ermöglicht, dass sich trotz der mit zunehmendem Aufpressdruck zunehmenden Verbiegung des distalen Endes des ersten Fixierstiftes oder gegebenenfalls der distalen Enden des ersten und/oder zweiten Fixierstiftes über eine möglichst große Fläche Last übertragen werden kann. Weiterhin wird es dadurch ermöglicht, dass die hauptlasttragenden Bereiche der sich ergebenden Kontaktflächen in Bezug auf den Fixierstift weiter nach radial außen verlagert werden, wodurch insbesondere in Bezug auf eine Rotationsbelastung um die entsprechende Belastungsachse ein erhöhter Klemmwiderstand erzeugt wird. Als approximierter Durchmesser wird dabei der Durchmesser der Kugel beziehungsweise des Kreises verstanden, die beziehungsweise der sich ergibt, wenn die sphärisch-konkave Krümmung solange erweitert würde, bis man wieder auf den Ausgangspunkt stieße.

Vorzugsweise ist der approximierte Durchmesser der sphärisch-konkav gekrümmten Kontaktfläche des ersten Fixierstiftes oder gegebenenfalls des ersten und/oder zweiten Fixierstiftes maximal 1,00 mm, vorzugsweise 0,50 mm, besonders vorzugsweise 0,20 mm und mindestens 0,01 mm, vorzugsweise 0,02 mm, besonders vorzugsweise 0,05 mm größer als der Durchmesser des als Kugelkopf ausgebildeten proximalen Endes des Knochenankers.

Vorzugsweise ist ein Federelement in der linearen Quer-Öffnung des Druckstückes angeordnet, und ist dieses Federelement insbesondere mittig in der linearen Quer-Öffnung platziert. Derart wird es ermöglicht, dass der erste und der zweite Fixierstift jeweils an dem Federelement vorbei in Eingriff mit dem Knochenanker führbar sind, ohne dass das in der linearen Quer-Öffnung des Druckstücks angeordnete Federelement dabei in kraftschlüssige Verbindung mit dem ersten und/oder zweiten Fixierstift tritt. Derart wird es ermöglicht, mittels des Fixier-Elementes eine sichere Vorfixierung des Knochenankers zu gewährleisten, ohne damit eine finale Fixierung des Knochenankers über das Verbindungselement zu erschweren beziehungsweise zu verhindern.

Vorzugsweise ist das in der linearen Quer-Öffnung angeordnete Federelement im distalen Bereich verbreiternd ausgebildet. Derart kann erreicht werden, dass bei eingesetztem Druckstück und in dessen Quer-Öffnung eingeführtem Fixier-Element, das Druckstück daran gehindert wird, ungewollt nach proximal hin aus dem Hauptkörper heraus auszutreten. Dabei fungiert das Fixier-Element zusätzlich als eine Verliersicherung für das Druckstück. Ferner wird dadurch die potentielle Kontaktfläche zwischen den Federelementen und dem proximalen Ende des Knochenankers maximiert, wodurch die Klemmwirkung der finalen Fixierung maximiert wird.

Vorzugsweise weist die Kontaktfläche des ersten Fixierstiftes oder gegebenenfalls des ersten und/oder zweiten Fixierstiftes eine lokal erhöhte Oberflächenrauigkeit auf. Derart kann die Reibwirkung und dadurch die Vorfixierwirkung erhöht werden. Die Oberflächenrauigkeit kann beispielsweise durch Aufrauhen oder durch das Vorsehen von Kerben und/oder Zähnen erzeugt werden.

Vorzugsweise weist das proximale Ende des Knochenankers eine lokal erhöhte Oberflächenrauigkeit auf. Derart kann die Reibwirkung und dadurch die Wirkung der Vorfixierung sowie der finalen Fixierung erhöht werden.

Vorzugsweise weist die Kontaktfläche des Druckstücks eine lokal erhöhte Oberflächenrauigkeit auf. Derart kann die Reibwirkung und dadurch die Wirkung der finalen Fixierung erhöht werden.

Der erste Aufnahmebereich ist als u-förmiger Gabelkopf ausgebildet, der einen ersten Gabelschenkel und einen zweiten Gabelschenkel aufweist. Derart wird eine gewichtsoptimierte und platzoptimierte Bauweise ermöglicht, wodurch beispielsweise die Handhabbarkeit des Befestigungssystems beziehungsweise der Osteosynthesevorrichtung insbesondere während der operativen Implantation in einen Patienten verbessert wird.

Vorzugsweise weist der erste Aufnahmebereich des Hauptkörpers ein Gewinde auf. Derart wird es ermöglicht, das Verbindungselement mittels einer Schraube, beispielsweise mittels einer platzsparenden Madenschraube, auf den Knochenanker, insbesondere mittelbar über das Druckstück auf den Knochenanker, zu pressen.

Ist der erste Aufnahmebereich als Gabelkopf ausgebildet, können beide Gabelschenkel einen entsprechenden Gewindeabschnitt aufweisen. Vorzugsweise weist dann der erste Gabelschenkel einen zu dem Gewindeabschnitt des zweiten Gabelschenkels komplementären Gewindeabschnitt auf, wobei beide Gewindeabschnitte zusammen das Gewinde des ersten Aufnahmebereiches des Hauptkörpers beziehungsweise des Gabelkopfes bilden. Ferner vorzugsweise sind die Gabelschenkel und damit auch die in diesen ausgebildeten Gewindeabschnitten einander gegenüberliegend angeordnet. Derart wird zum einen ein sicheres Festschrauben des Verbindungselementes ermöglicht und zum anderen eine weitere Gewichts- und Bauraumreduktion.

Vorzugsweise sind der dritte Aufnahmebereich und die Axial-Öffnung an dem ersten Gabelschenkel angeordnet. Derart kann der benötigte Bauraum reduziert werden, wodurch die Handhabbarkeit verbessert wird.

Vorzugsweise folgt von proximal nach distal nach dem distalen Ende der Axial-Öffnung das proximale Ende des dritten Aufnahmebereiches des Hauptkörpers. Derart kann der notwendige Bauraum reduziert werden. Ferner wird es dadurch ermöglicht, das Fixier-Element von radial außen nach radial innen in die Quer-Öffnung des Hauptkörpers und gegebenenfalls in die Quer-Öffnung des Druckstücks einzusetzen, wodurch die Handhabbarkeit verbessert wird.

Vorzugsweise ist die Axial-Öffnung derart ausgebildet und positioniert und/oder das Stell-Element derart ausgebildet, dass das Stell-Element derart positionierbar ist, dass dessen distales Ende derart nach distal hin aus der Axial-Öffnung ausstehen kann, dass das radial äußere Ende der Quer-Öffnung des Hauptkörpers nach radial außen hin verdeckt ist. Dadurch kann mittels des Stell-Elementes für das Fixier-Element nach radial außen hin eine Verliersicherung erreicht werden.

Eine erfindungsgemäße Osteosynthesevorrichtung, insbesondere zur Wirbelsäulenbehandlung, weist einen Knochenanker und ein erfindungsgemäßes Befestigungssystem auf.

Eine alternative Osteosynthesevorrichtung, insbesondere polyaxiale Pedikelschraube besteht aus einem Kopf aufweisenden Knochenanker, einem in einer Seitenansicht u-förmigen Gabelkopf, einem darin befindlichen Druckstück, einem innenliegenden und in einer Quer-Öffnung geführtem Fixier-Element, einem lösbar verbundenen Stell-Element zur Betätigung des Fixier-Elements, wobei das Fixier-Element dazu geeignet ist, den Knochenanker-Kopfbereich temporär in allen Freiheitsgraden zu klemmen, ohne dass dabei ein Druckstück notwendig ist, oder falls vorhanden, nicht belastet wird.

In der bevorzugten Ausgestaltungsform ist es möglich, den Klemmeffekt mittelbar über ein Stell-Element beziehungsweise unmittelbar über das Fixier-Element einzuleiten ohne dass der Verbindungsstab oder die Madenschraube vorhanden sind. Da es sich nicht um eine finale Klemmung mit eingelegtem Verbindungsstab handelt, nennt sich diese Art der Klemmung, temporäre Klemmung. Mit der temporären Klemmung ist es für den Anwender möglich, während der Operation eine polyaxiale Schraube in einer gewünschten Winkelstellung in eine monoaxiale Schraube zu konvertieren. Das heißt, es sind alle rotatorischen Freiheitsgrade einer polyaxialen Schraube temporär gesperrt. Die Schraube verhält sich monoaxial. Damit kann der Anwender nun den zu behandelten Wirbel translatorisch als auch rotatorisch so lange manipulieren, bis er in der gewünschten Endstellung einen Verbindungsstabe einlegt und mit der Madenschraube fixiert. Mit einer polyaxialen Schraube sind solche Korrektionsmanöver nicht möglich, da eine von

Patienten-seitig außen initiierte Korrektionsmanöver in einer freien Bewegung des polyaxial- Kugel-Gelenks resultiert und somit nicht an den Wirbel weitergeleitet wird. Dies funktioniert nur mit deaktivierten rotatorischen Freiheitsgraden im Kugelgelenk, also temporär geklemmt ist.

Unabhängig von der temporären Klemmung, muss nach der Implantation der Osteosynthesevorrichtung in den Knochen, ein Verbindungsstab eingelegt und mit Hilfe eines Verschlusselements die Osteosynthesevorrichtung final in allen Freiheitsgraden fixiert werden. Dies geschieht durch Festschrauben des Verschlusselements. Bei fest angezogenen Verschlusselement wird eine axiale Kompressionskraft vom Verschlusselement auf den Verbindungsstab übertragen, und dieser drückt auf die Stabauflagerstellen des Druckstücks und erzeugt eine minimale Relativbewegung des Druckstücks weiter nach distal, so dass der Knochenanker winkelstabil im Kugelsitz geklemmt wird. Optimalerweise werden zwei oder mehr Osteosynthesevorrichtungen mit Hilfe eines Verbindungsstabs miteinander verbunden.

Dabei ist der Gabelkopf so ausgestaltet, dass ein Verbindungsstab einlegbar und mit einem Verriegelungs-Element am Gabelkopf fixierbar ist. Dadurch wird wie bereits erwähnt eine winkelstabile Klemmung zwischen dem Knochenanker-Kopfbereich und Gabelkopf erzielt. Die winkelstabile Klemmung mit Hilfe des Verriegelung-Elements und die winkelstabile Klemmung mit Hilfe des Fixier-Elements funktionieren in dem erfindungsgemäßen Aufbau unabhängig voneinander. Sie sind getrennt aber auch in Kombination aktivierbar.

In einer bevorzugten Ausführungsform der Osteosynthesevorrichtung besitzt der Gabelkopf eine Durchgangsöffnung und formt in proximaler Richtung zwei Gabelschenkel mit einem innenliegenden Gewinde für ein Verriegelungs-Element aus. In distaler Richtung ist in der Durchgangsöffnung ein Kugelkopfaufnahmebereich vorgesehen, worin ein Knochenanker schwenkbar gelagert ist.

Als Knochenanker kommen vorzugsweise Knochenschrauben zum Einsatz, die mit einem Knochen verschraubbar sind. Es sind aber auch Haken, klingenähnliche Anker, Klemmen, Nägel und andersartig gestaltete Knochenanker anwendbar. Die wesentlichen Merkmale des Knochenankers sind ein kugelähnlicher Kopf, ein Halsbereich und ein Bereich, welcher im oder am Knochen verankert oder befestigt werden kann. In dieser Patentanmeldung soll hauptsächlich auf den Gabelkopf eingegangen werden, und mit Knochenanker sollen alle erdenklichen mit einem Knochen verbindbaren Elemente verstanden werden.

Ein wesentliches Merkmal ist, dass das Zentrum des innenliegenden Gewindes und das Zentrum des Kugelkopfaufnahmebereichs die Position und Orientierung der Zentralachse definiert. Seitlich und in einem Abstand, der größer ist als der Durchmesser des Verriegelung-Elements, ist eine Axial-Öffnung für das Stell-Element vorgesehen. Die Axial-Öffnung ist hauptsächlich parallel zur Zentralachse angeordnet. Damit kann das Stell-Element aus derselben Richtung, wie das Verriegelung-Element, mit Hilfe von Instrumenten angetrieben werden. In der Axial-Öffnung wird das Stell-Element längenverstellbar geführt. Mit dem Begriff Axial-Öffnung sind, neben vollständig von Material umschlossene Öffnungen, auch Teil-Öffnungen oder Teil-Ausschnitte wie beispielsweise c-förmige Ausschnitte quer zur Zentralachse (103) gemeint.

Quer zu der Axial-Öffnung und der Zentralachse ist eine zusätzliche Öffnung, eine Quer-Öffnung, vorgesehen. Die Quer-Öffnung stellt eine Verbindung zwischen Axial-Öffnung und Durchgangs-Öffnung des Gabelkopfs her. In dieser Quer-Öffnung ist das Fixier-Element entlang der Quer-Öffnungs-Achse beweglich geführt. Mit Einleitung einer Kompressionskraft entlang der Quer-Öffnungs-Achse klemmt das Fixier-Element den Kopfbereich des Knochenankers winkelstabil im Gabelkopf.

Die temporäre Klemmung bzw. die Kompressionskraft wird vorzugsweise durch ein Stell-Element, welches in der Axial-Öffnung geführt ist durch Verstellung des Stell-Elements erzeugt. Die Kompressionskraft wird dabei auf das Fixier-Element übertragen bzw. umgelenkt, so dass daraus eine Kompressionskraft entlang der Quer-Öffnungs-Achse resultiert, die zur temporären Klemmung des Knochenanker-Kopfbereichs im Gabelkopf führt.

Alternativ ist es auch denkbar, dass die temporäre Klemmung bzw. die Einleitung einer Kompressionskraft über das Fixier-Element selbst erfolgen kann. Hierfür wäre es beispielsweise erforderlich, dass das Fixier-Element und die Quer-Öffnung mit Hilfe eines Gewindeabschnitts miteinander im Eingriff stehen und so eine Längenverstellung ermöglicht wird.

Ein charakteristisches Merkmal der Osteosynthesevorrichtung ist, dass die Quer-Öffnungs-Achse des Fixier-Elements ungefähr auf das virtuelle Zentrum des Kugelkopfaufnahmebereichs gerichtet ist und sich mit der Zentralachse schneidet. Dadurch lässt sich die Kompressionskraft auf den Schwenk-Mittelpunkt des Knochenankers im Gabelkopfs richten und eine optimale Klemmung erzielen.

Optimalerweise stellt der Gabelkopf eine seitliche Aussackung oder Materialaufdickung bereit, um darin die Axial-Öffnung, Quer-Öffnung und die zur Betätigung notwendigen Elemente (Stell- und Fixier-Element) aufzunehmen.

In einer bevorzugten Ausführungsform verfügt der Gabelkopf über ein Druckstück, welches einen nach distal gerichteten Kontakt-Bereich zum Knochenanker-Kopfbereich und ein nach proximal gerichtetes Stablager ausweist. Im Bereich des Knochenanker-Kopfbereichs ist eine seitliche Öffnung oder ein teilweiser Ausschnitt vorgesehen, worin das Fixier-Element frei beweglich angeordnet ist. Dadurch ist eine Aktivierung des Fixier-Elements ohne die Belastung des Druckstücks ermöglicht.

In einer bevorzugten Ausführungsform kann der Gabelkopf von distal kommend mit Knochenankern montiert werden. Durch diese vorteilhafte Anordnung der Komponenten können die Knochenanker durch Aufsetzen bzw. Aufpressen relativ einfach mit dem Gabelkopf montiert werden. Durch ein Hilfsmittel, wie beispielsweise einem Löse-Instrument, ist der Knochenanker auch wieder entfernbar. Damit kann die erfindungsgemäße Osteosynthesevorrichtung modular vom Anwender konfiguriert und im Operationssaal zu einem späteren Zeitpunkt als der der Fertigung, zusammengesetzt werden. Dadurch ist es beispielsweise möglich, dass zuerst der Knochenanker einzeln in den Knochen verankert oder geschraubt wird, und dann im Anschluss der Gabelkopf am bereits implantierten Knochenanker befestigt wird. Dies hat den großen Vorteil, dass der Chirurg nach der Implantation des Knochenankers deutlich mehr im Platz und eine bessere Sicht im OP-Feld im Vergleich zu den sonst vollständig implantierten Pedikelschrauben hat.

Vorteil ist, dass einerseits größere Knochenanker, d.h. Knochenanker mit einem größeren Außendurchmesser, als der distale Innendurchmesser des Gabelkopfes montiert werden können. Andererseits kann das Knochenanker-Portfolio minimalisiert werden, da der Anwender Gabelkopf und Knochenanker während der Operation miteinander kombinieren kann, anstatt auf ein vorgefertigtes übergroßes Portfolio zurückzugreifen. Ein solches Portfolio muss beim Anwender vorrätig sein und somit ist deutlich mehr Kapital gebunden, als es die erfindungsgemäße modulare Version erfordern würde.

Für eine modulare Gestaltung der Osteosynthesevorrichtung ist es vorteilhaft, wenn das Druckstück in distaler Richtung offene Schlitze aufweist und dadurch wenigstens drei federelastische Arme am Kopfaufnahmebereich ausgebildet sind. Die federelastische Arme können nach radial außen auslenken und somit den Knochenanker-Kopfbereich umschließen. Dadurch kann ein Knochenanker von distaler Richtung kommend in das Druckstück eingeklippt werden. Das Druckstück formt am distalen Ende der Außenseite wenigstens abschnittsweise einen Konus. Im Gabelkopf auf Höhe des Kugelaufnahmebereichs ist wenigstens ein Innen-Konus-Abschnitt definiert, welche kongruent zum Konus des Druckstücks und bei Betätigung des Verriegelung-Elements zur winkelstabilen Klemmung des Knochenanker-Kopfbereichs mit dem Gabelkopf führt. Auch hier ist es wesentlich, dass das Druckstück eine Durchführungsöffnung für das Fixier-Element vorsieht, damit das Druckstück bei aktivierter temporärer Klemmung nicht belastet wird.

Am proximalen Gabelkopfbereich ist eine umlaufene Nut mit einem hakenähnlichen Profil vorgesehen, die einen Hintergriff für ein Instrument bereitstellt. Stellvertretend sind andersartig gestaltete Nutprofile oder auch andere Haltemerkmale wie z.B. Öffnungen denkbar, die einen Hintergriff für ein Instrument bereitstellen.

Am proximalen Ende des Gabelkopfs können sich weitere und lösbare Abschnitte mit einem Gewindebereich befinden, die eine Reposition des Verbindungsstabs erlauben. Es ist auch denkbar, dass ein durch zwei längere Schenkel ausgebildeter hülsenähnlicher Zugang vorgesehen ist, wie es für den minimal-invasiven Zugang zum Einsatz kommt. Dabei können die lösbaren Schenkelverlängerungen optional am proximalen Ende miteinander verbunden sein. Mit lösbarer Verbindung sind beispielsweise Sollbruchstellen gemeint, die dazu geeignet sind, die Verlängerungen nach final fixiertem Verbindungsstab zu entfernen.

Als Material eignen sich alle metallischen Legierungen, die als orthopädischer Implantatwerkstoff bekannt und akzeptiert sind. Dazu gehören beispielsweise Titan-, Cobalt-Chrom und Edelstahllegierungen. Sollte die herkömmliche Herstellung des Gabelkopfes und des Verriegelungsrings nicht oder nur unter höchsten technologischen Aufwand möglich sein, stellt die additive Fertigung das Mittel der Wahl dar. Additive Fertigungen von metallischen Legierungen, oder auch 3D-Druck genannt, greifen auf das Laser- oder Elektronenstrahl-Schmelzverfahren zurück.

### Kurze Beschreibung der Figuren

Bei den in den Figuren 1a-9 gezeigten Beispielen handelt es sich nicht um Ausführungsbeispielen der Erfindung. Sie dienen lediglich dem Verständnis der Erfindung.
Fig. 1a zeigt eine Schrägansicht einer Osteosynthesevorrichtung.
Fig. 1b zeigt eine Schrägansicht der Osteosynthesevorrichtung mit gelöstem Stell-Element.
Fig. 2 zeigt eine Explosionsdarstellung der Osteosynthesevorrichtung bestehend aus einem Gabelkopf, einem Fixier-Element, Knochenanker und Druckstück.
Fig. 3 zeigt eine Seitenansicht der montierten Osteosynthesevorrichtung mit Schnittdarstellung.
Fig. 4 zeigt eine Illustration der für die Betätigung der temporären Klemmung erforderlichen Komponenten.
Fig. 5a, b zeigt zwei unterschiedliche Stellungen S1, S2 des Stell-Elements.
Fig. 6a zeigt eine Schrägansicht und Fig. 6b die dazu gehörige Explosionsdarstellung einer alternativen Ausgestaltungsform, bei der eine Montage des Knochenankers aus distaler Richtung kommend möglich ist.
Fig. 7 stellt eine Seitenansicht der montierten Osteosynthesevorrichtung aus Fig. 6a und 6b mit entsprechender Schnittdarstellung vor.
Fig. 8 zeigt die fertig implantierte Osteosynthesevorrichtung in einer Schrägansicht.
Fig. 9 zeigt eine alternative Ausgestaltungsform einer implantierten Osteosynthesevorrichtung, bei der das Stell-Element im Patienten verbleibt.
Fig. 10a zeigt ein erfindungsgemäßes Befestigungssystem in einer isometrischen Ansicht.
Fig. 10b zeigt eine Osteosynthesevorrichtung mit dem in Fig. 10a dargestellten Befestigungssystem in einer isometrischen Ansicht.
Fig. 11 zeigt das in den Fig. 10a und 10b dargestellte Befestigungssystem in einer Explosionsansicht.
Fig. 12a zeigt die in Fig. 10b dargestellte Osteosynthesevorrichtung in einer Seitenansicht mit einer Schnittebene.
Fig. 12b zeigt die in den Fig. 10b und 12a dargestellte Osteosynthesevorrichtung in einer Schnittansicht entlang der ebenfalls in Fig. 12a dargestellten Schnittebene.
Fig. 13a zeigt ein Fixier-Element mit einem ersten Fixierstift und einem zweiten Fixierstift in einer isometrischen Ansicht.
Fig. 13b zeigt das in Fig. 13a dargestellte Fixier-Element in einer Seitenansicht.
Fig. 13c zeigt das in den Fig. 13a und 13b dargestellte Fixier-Element in einer Aufsicht.
Fig. 13d zeigt das in den Fig. 13a bis 13c dargestellte Fixier-Element von unten.
Fig. 14a zeigt ein Stell-Element in Eingriff mit dem in den Fig. 13a bis 13d dargestellten Fixier-Element und einem Knochenanker in einer isometrischen Ansicht.
Fig. 14b zeigt ein Fixier-Element mit einem ersten Fixierstift im Eingriff mit einem Kopfbereich eines Knochenankers in einer Aufsicht.
Fig. 14c zeigt das in den Fig. 13a bis 13d dargestellte Fixier-Element in der in Fig. 14a dargestellten Eingriffsstellung mit dem Kopfbereich des Knochenankers in einer Aufsicht.
Fig. 15 zeigt das in Fig. 10a dargestellte Befestigungssystem in einer anderen isometrischen Ansicht.
Fig. 16a zeigt das in den Fig. 13a bis 13d dargestellte Fixier-Element im Eingriff mit einem Druckstück in einer isometrischen Ansicht.
Fig. 16b zeigt das in Fig. 16a dargestellte Fixier-Element in der ebenfalls in Fig. 16a dargestellten Eingriffstellung mit dem Druckstück in einer Seitenansicht mit einer Schnittebene.
Fig. 16c zeigt das in den Fig. 16a und 16b dargestellte Fixier-Element in der ebenfalls in den Fig. 16a und 16b dargestellten Eingriffstellung mit dem Druckstück in einer Schnittansicht entlang der ebenfalls in Fig. 16b dargestellten Schnittebene.
Fig. 17 zeigt die in den Fig. 10b, 12a und 12b dargestellte Osteosynthesevorrichtung mit einem Verbindungselement in einer isometrischen Ansicht.

### Beschreibung der bevorzugten Ausführungsformen

Wird im Folgenden mittels Bezugszeichen auf konkret in den Figuren dargestellte Merkmale Bezug genommen, ist die in diesem Zusammenhang beschriebene erfinderische Lehre nicht als auf die jeweils konkret dargestellte Ausführungsform beschränkt zu verstehen. Vielmehr ist die folgende Beschreibung unter Berücksichtigung der vorstehenden allgemeinen Grundsätze der erfinderischen Lehre auszulegen.

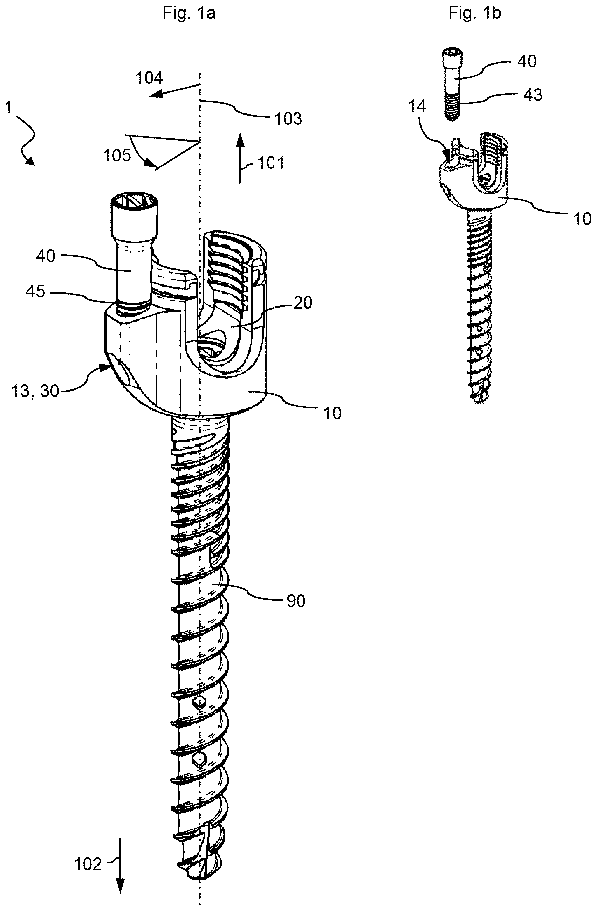

Beschrieben wird eine Osteosynthesevorrichtung 1, zur Behandlung der Wirbelsäule, wobei mehr als eine Osteosynthesevorrichtung 1 eingesetzt wird, um ein oder mehr Wirbel mit Hilfe von Verbindungsstäben 50 miteinander zu verbinden und somit die Wirbelsäule zu stabilisieren. Für die Osteosynthesevorrichtung 1, insbesondere für den Gabelkopf 10 sind raumzuweisende Koordinatenreferenzen definiert, wie zum Beispiel die proximale Richtung 101, die distale Richtung 102, die sich entlang einer Zentralachse 103 ausdehnen. Von der Zentralachse 103 nach außen abgehend definiert die radiale Ausbreitung 104 und die umfängliche Ausbreitung 105 ist durch einen konstanten Radius und einen variablen Umfangswinkel definiert (Fig. 1).

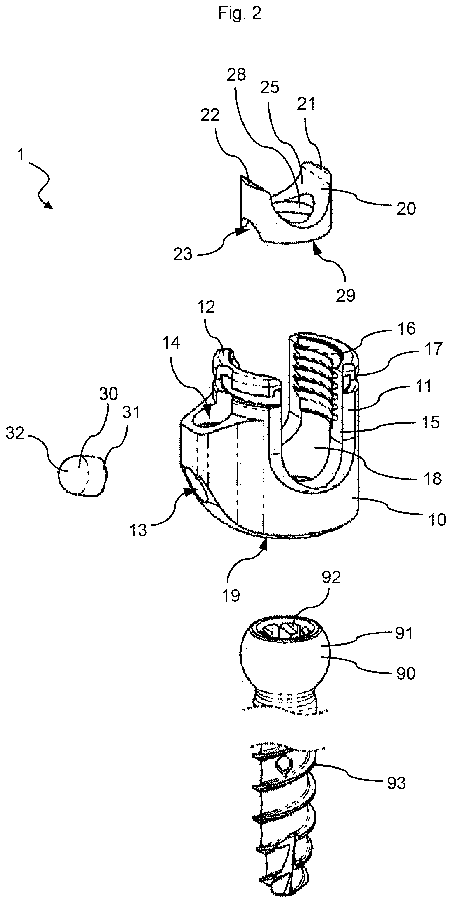

Fig. 1a, 1b und Fig. 2 zeigen die Osteosynthesevorrichtung 1, zur Behandlung der Wirbelsäule, bestehend aus einem in einer Seitenansicht u-förmigen Gabelkopf 10, welcher eine Durchgangsöffnung 18 besitzt und der Gabelkopf 10 in proximaler Richtung 101 zwei Gabelschenkel 11, 12 mit einem innenliegenden Gewinde 16 aufweist, und darin ein Verbindungsstab 50 aufgenommen werden kann, und im Gabelkopf 10 in distaler Richtung 102 in der Durchgangsöffnung 18 ein Kugelkopfaufnahmebereich 19 vorgesehen ist und darin ein Knochenanker 90 schwenkbar gelagert ist, wobei das Zentrum des innenliegenden Gewindes 16 und das Zentrum des Kugelkopfaufnahmebereichs 19 eine Zentralachse 103 definieren und wenigstens ein Schenkel 11, 12 eine zur Zentralachse 103 parallel angeordnete Axial-Öffnung 14 für ein Stell-Element 40 bereitstellt. Im Gabelkopf 10 ist eine Quer-Öffnung 13 vorgesehen, welche mit der Axial-Öffnung 14 und mit der Durchgangsöffnung des Gabelkopfs 18 kommuniziert. In dieser Quer-Öffnung 13 ist ein Fixier-Element 30 entlang einer Achse 130 beweglich geführt. Mit Einleitung einer Kompressionskraft entlang der Quer-Öffnungs-Achse 130 klemmt das Fixier-Element 30 den Kopfbereich 91 des Knochenankers 90 winkelstabil im Gabelkopf 10. Mit Entlastung der Kompressionskraft am Fixier-Element 30 wird der Kopfbereich des Knochenankers 91 im Gabelkopf 10 wieder beweglich.

Der Knochenanker 90 weist vorzugsweise einen Kopfbereich 91 mit einer darin befindlichen Werkzeugansatzstelle 92 auf und besitzt in distaler Richtung 102 ein Knochengewinde 93.

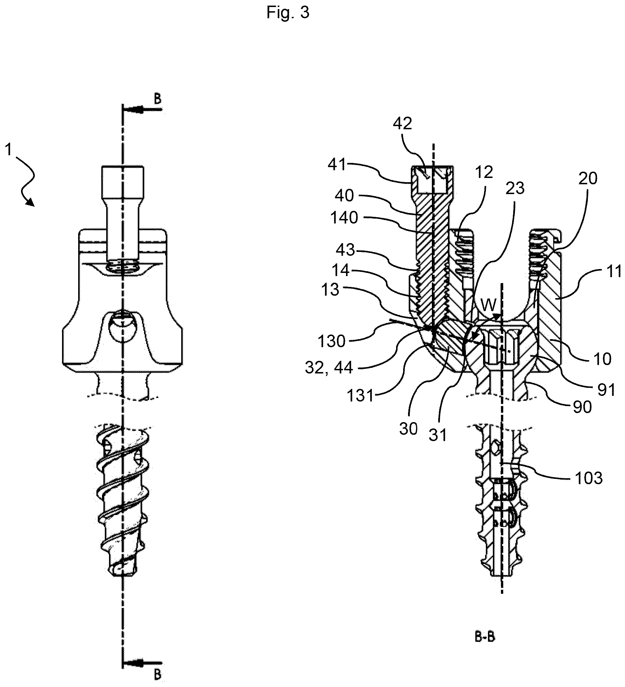

Die Osteosynthesevorrichtung 1 besitzt vorzugsweise ein stiftförmiges Stell-Element 40, welches in proximaler Richtung 101 eine Werkzeugansatzstelle aufweist 42 (Fig. 3). Das Stell-Element 40 ist lösbar gestaltet und kann ggf. auch entfernt werden (Fig. 1b). Mit entferntem Stell-Element 40 und nicht temporär geklemmten Fixier-Element 30 verhält sich diese Osteosynthesevorrichtung 1 wie eine polyaxiale Pedikelschraube.

Fig. 1a und Fig. 2 illustrieren auch, dass der Gabelkopf 10 der Osteosynthesevorrichtung 1 über ein Druckstück 20 verfügt und dass das Druckstück eine Durchgangsöffnung 28, einen nach distal gerichteten Kontakt-Bereich zum Knochenanker-Kopfbereich 29, ein nach proximal gerichtetes Stablager 25, welches durch zwei Schenkel 21, 22 abgegrenzt ist und im Bereich des Knochenanker-Kopfbereichs 29 eine seitliche Öffnung oder teilweisen Ausschnitt 23 aufweist, worin das Fixier-Element 30 frei beweglich angeordnet ist. Wird ein Verbindungsstab 50 in die u-förmige Gabelöffnung 15 eingelegt, steht der Verbindungsstab 50 mit dem Stablager des Druckstücks 25 in direktem Kontakt. Wird nun das Verriegelungs-Element 60 mit dem Gabelkopf 10 fixiert, wird eine Kompressionskraft vom Verriegelungs-Element 60 auf den Verbindungsstab 50, und von diesem auf das Druckstück 20, 25 und vom Druckstück 20 auf den Knochenanker-Kopfbereich 29, 91, und der Knochenanker 90 gegen den distalen Kugelsitzbereich 19 übertragen. Diese eingeleitete Kompressionskraft führt dann zur Klemmung der Polyaxialität.

Fig. 2 zeigt den bevorzugten Aufbau des Fixier-Elements 30. Hier dargestellt ist, dass das Fixier-Element 20 vorzugsweise als Rundstab gestaltet und in einer konzentrischen Quer-Öffnung 13 beweglich gelagert ist. Vorstellbar sind auch alternative aber hier nicht gezeigte Ausführungen des Fixier-Elements 20, wie beispielsweise, dass das Fixier-Element in einer Schnittdarstellung quer zur Quer-Öffnungs-Achse 130 als Dreieck, Viereck oder Vieleck, oder auch als Drei- oder Vieleck mit Übergangsrundungen ausgestaltet ist, oder als Vollrund mit seitlichen Abflachungen vorgesehen ist, die dazu dienen, dass eine Rotation des Fixier-Elements 30 unterbunden wird. Denkbar ist auch, dass das Fixier-Element 30 entlang der Quer-Öffnungs-Achse 130 seine Geometrie ändert, um Eingriffsmerkmale für Verliersicherungs-Elemente bereitzustellen.

Der Fig. 2 ist ebenfalls zu entnehmen, dass der Gabelkopf 10 Vorsprünge, Öffnungen, Nuten, Stege, Profile oder sonstige Merkmale 17 die zum Er-, Ein- oder Hintergreifen mit einem Instrument geeignet sind, besitzt. Wenn das Stell-Element 40 Teil eines Instruments ist kann dieses Instrumenten-Anbindungsmerkmal 17 dazu dienen eine Zugkraft am Gabelkopf 10 einzuleiten, welche als Antagonist für das Einleiten einer Kompressionskraft über das Stell-Element 40 wirkt.

Fig. 2 zeigt auch, wenn eine Axial-Öffnung 14 in einem Gabelkopf-Schenkel 11, 12 vorgesehen ist, dass es vorteilhaft ist, dass der Gabelkopf 10 eine seitliche Materialwulst besitzt, welche in einen der Schenkel 11 oder 12 übergeht, damit die Axial-Öffnung 14 überhaupt herstellbar ist.

Fig. 3 zeigt die Osteosynthesevorrichtung 1 in einer Seitenansicht und im Schnitt. Erkennbar ist, dass die Achse des Fixier-Elements 130 ungefähr auf das virtuelle Zentrum des Kugelkopfaufnahmebereichs 19 gerichtet ist und sich mit der Zentralachse 103 schneidet. Außerdem schneidet sich die Quer-Öffnungs-Achse 130 auch mit der Axial-Öffnungs-Achse für das Stell-Element 140. Somit ist die Kompressionskraft auf den Mittelpunkt der kugelähnlichen Knochenanker-Kopfbereich 91 ausgerichtet, so dass auch bei verschwenkten Knochenanker 90 ein ähnlich-großer Kontakt-Bereich 31, 91 vorliegt. Des Weiteren ist es vorteilhaft, wenn die Achse des Fixier-Elements 130 im Bezug zur Zentralachse 103 in einem Winkel W zwischen 10° und 90°, vorzugsweise zwischen 30° und 85°, vorzugsweise zwischen 60° und 80° angeordnet ist. Dadurch wird erreicht, dass bei eingeleiteter Kompressionskraft durch das Fixier-Element 30 der Knochenanker-Kopfbereich 91 nicht nur in die gegenüberliegende Richtung bzw. an die gegenüberliegende Innenwandung des Gabelkopfs 18 gedrückt wird, sondern auch anteilsweise in den Kugelsitz 19 gezwungen wird. Somit wird der Knochenanker 90 im Gabelkopf 10 im Kugelsitz 19 unter Krafteinwirkung zentriert.

Fig. 2 zeigt auch, dass die Öffnung des Fixier-Elements 13 entlang der Fixier-Element-Achse 130 wenigstens an einer Stelle einen Absatz, Hinterschneidung, einen Stift, Stufe, verformbaren Bereich oder Verjüngung 131 besitzt, die dazu geeignet ist, das Fixier-Element 30 verliersicher in dieser Öffnung 13 zu halten. Für die Montage der Osteosynthesevorrichtung 1 wird das Fixier-Element 13 von der zentralen Durchgangsöffnung 18 kommend in die Quer-Öffnung 13 gesteckt. Im Anschluss wird der Knochenanker 90 mit seinem Kopfbereich 91 in die Durchgangsöffnung 18 in den Kugelsitz 19 platziert und wird so das Fixier-Element 30 in der Quer-Öffnung 13 verliersicher gehalten.

Alternativ ist es auch denkbar, dass die Quer-Öffnung 13 für das Fixier-Element 30 wenigstens abschnittsweise ein Innengewinde besitzt, so dass das Fixier-Element 30 durch Rotation selbst die Kompressionskraft erzeugen kann. Alternativ kann ein abschnittsweises Innengewinde auch als Verliersicherung des Fixier-Elements 30 dienen, indem das Gewinde eine zu überbrückende Barriere darstellt.

Die Osteosynthesevorrichtung 1 ist so ausgestaltet, dass ein Verbindungsstab 50 einlegbar und mit einem Verriegelungs-Element 60 am Gabelkopf 10 fixierbar ist, und dadurch eine winkelstabile Klemmung zwischen Knochenanker-Kopfbereich 91 und Gabelkopf 10 erzielt wird, und die winkelstabile Klemmung mit Hilfe des Verriegelung-Elements 60 und die winkelstabile Klemmung mit Hilfe des Fixier-Elements 30 unabhängig voneinander aktivierbar und auch miteinander kombinierbar sind.

In Fig. 3 ist auch das wesentliche charakteristische Merkmal zu erkennen, nämlich dass bei temporärer Klemmung des Knochenanker-Kopfbereichs 91 alleinig durch das Fixier-Element 30 das Druckstück 20 stets unbelastet ist. Erst durch Einlegen und Fixieren eines Verbindungsstabs 50, 60 wird das Druckstück mit einer Kraft beaufschlagt. Dadurch kann der Gabelkopf und das Druckstück eine höhere mechanische Belastung aufnehmen und das Druckstück kann mit deutlich weniger Material als vergleichbare Konzepte vorgesehen werden, was sich wiederrum positiv auf die Bauhöhe der Osteosynthesevorrichtung 1 auswirkt. Bei Osteosynthesevorrichtungen 1 für die Wirbelsäule ist eine kleinstmögliche Bauhöhe wichtig, um sich bestmöglich den anatomischen Gegebenheiten des Patienten anzupassen.

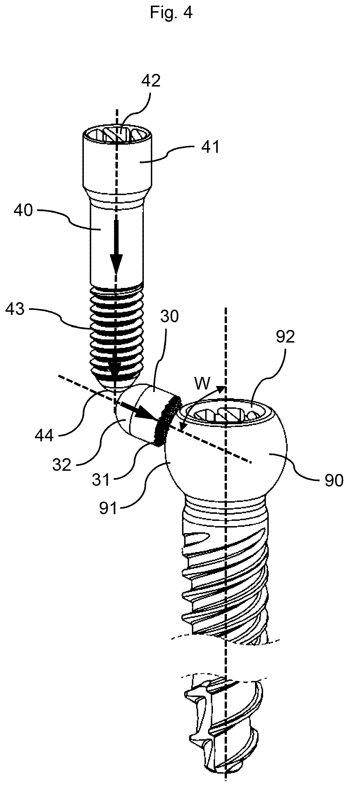

In Fig. 4 zeigt einen reduzierten Aufbau, bei dem nur die zur temporären Klemmung erzeugenden Elemente eingeblendet sind. Die restlichen Elemente wurden ausgeblendet. Durch Verstellung des Stell-Elements 40 kann eine Kompressionskraft erzeugt werden, welche auf das Fixier-Element 30 übertragen bzw. umgelenkt wird, und dadurch wird eine Kompressionskraft entlang der Quer-Öffnungs-Achse 130 erzeugt, die zur temporären Klemmung des Knochenanker-Kopfbereichs 91 im Gabelkopf 10 führt.

Dabei wird das Stell-Element 40 in der Axial-Öffnung 14 des hier ausgeblendeten Gabelkopfs 10 geführt. Ist das Stell-Element 40 Teil des Implantats ist es vorteilhaft, wenn die Axial-Öffnung 14 für das Stell-Element 40 wenigstens abschnittsweise ein Innengewinde besitzt. Damit im Eingriff stehend ist es erforderlich, dass das Stell-Element 40 selbst auch wenigstens abschnittsweise ein Gewinde 43 besitzt. Dadurch ist es möglich, dass bei eingeschraubtem bzw. angezogenem Stell-Element 40 die eingeleitete Kompressionskraft aufrechterhalten bleibt. Ist das Stell-Element 40 Teil eines Instruments muss das Stell-Element 40 selbst kein Gewinde aufweisen, da die einzuleitende Kompressionskraft durch das Instrument generiert wird.

Für die Umlenkung der Kompressionskraft vom Stell-Element 40 auf das Fixier-Element 30 ist es vorteilhaft, wenn das Fixier-Element 30 einen nach radial außen gerichteten Kontakt-Bereich 32 aufweist, welcher in direktem Kontakt mit einem distalen Kontakt-Bereich 44 des Stell-Elements 40 steht und dieser Kontakt 32, 44 so ausgestaltet ist, dass eine Kompressionskraft entlang der Stell-Element-Achse 140 in eine Kompressionskraft entlang der Fixier-Element-Achse 130 umgeleitet wird. Dabei kann der nach radial außen gerichteten Kontakt-Bereich 32 des Fixier-Elements 30 in wenigstens einer Seitenansicht abschnittsweise konvex vorgesehen sein (Fig. 4). Alternativ ist es auch denkbar, dass der nach radial außen gerichteten Kontakt-Bereich 32 des Fixier-Elements 30 in wenigstens einer Seitenansicht eine Schräge aufweist. In der bevorzugten Ausführungsform ist es so vorgesehen, dass der distale Kontakt-Bereich 44 des Stell-Elements 40 wenigstens abschnittsweise konvex ausgestaltet ist.

Der für die temporäre Klemmung verantwortliche nach radial innen gerichteten Kontakt-Bereich 31 des Fixier-Elements 30, liegt unmittelbar am Kopfbereich 91 des Knochenankers 90 an. Vorteilhaft ist es, wenn dieser Kontakt-Bereich 31 in einer Seitenansicht wenigstens abschnittsweise konkav ausgeformt ist oder wenigstens einen Abschnitt der Außenfläche des Knochenanker-Kopfbereichs 91 approximiert. Für eine optimierte Klemmwirkung ist es vorteilhaft, wenn der nach radial innen gerichtete Kontakt-Bereich 31 des Fixier-Elements 30 eine erhöhte Rauigkeit, Kerben oder Zähne aufweist (Fig. 4).

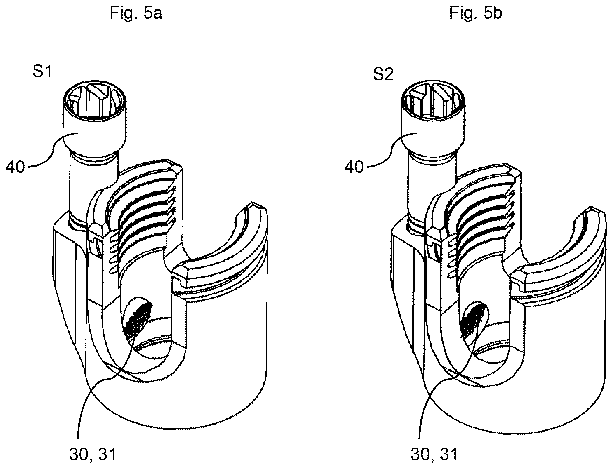

Fig. 5 ist zu entnehmen, dass das Fixier-Element 30 entlang der Quer-Öffnungs-Achse 130 eine Stellung S1 einnehmen kann, bei der eine Kompressionskraft auf den Knochenanker 90 übertragen wird, so dass Knochenanker 90 winkelstabil im Kugelsitz 19 gehalten wird und das Fixier-Element 30 eine zweite Stellung S2 einnehmen kann, bei der der Knochenanker 90 beweglich im Kugelsitz 19 gehalten wird. Eingestellt werden können diese Stellungen des Fixier-Elements 30 durch eine Verstellung des Stell-Elements 40. Dabei existiert eine Stellung S1 bei der das Stell-Element 40 das Fixier-Element 30 zur temporären Klemmung nach radial innen verfahren und eine Stellung S2 des Stell-Elements bei der das Fixier-Element so verstellt oder nach radial außen verfahren ist, dass sich eine freie Polyaxialität des Knochenankers ergibt.

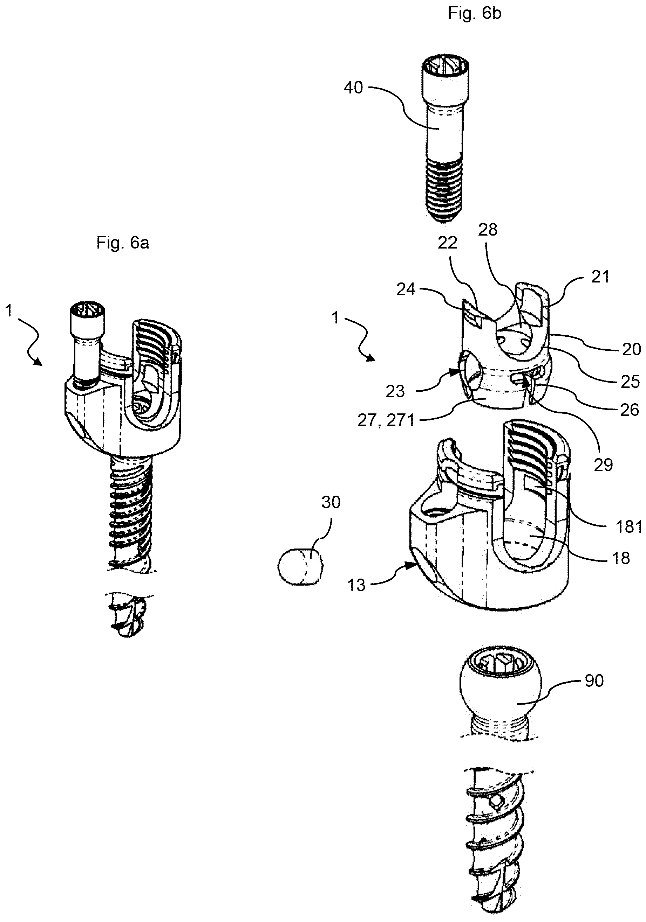

In Fig. 6a und 6b ist eine weitere Osteosynthesevorrichtung 1 gezeigt. Hier weist das Druckstück 20 in distaler Richtung 102 offene Schlitze 26 auf. Dadurch sind wenigstens drei federelastische Arme 27 am Kopfaufnahmebereich 29 ausgebildet, wobei die federelastische Arme 27 an ihrer Außenseite wenigstens abschnittsweise einen Konus 271 beschreiben, und der Knochenanker 90 von distaler Richtung 102 kommend in den Gabelkopf 10 gesteckt werden kann. Dadurch können Knochenanker 90 mit größerem Außendurchmesser mit dem Gabelkopf 10 montiert werden.

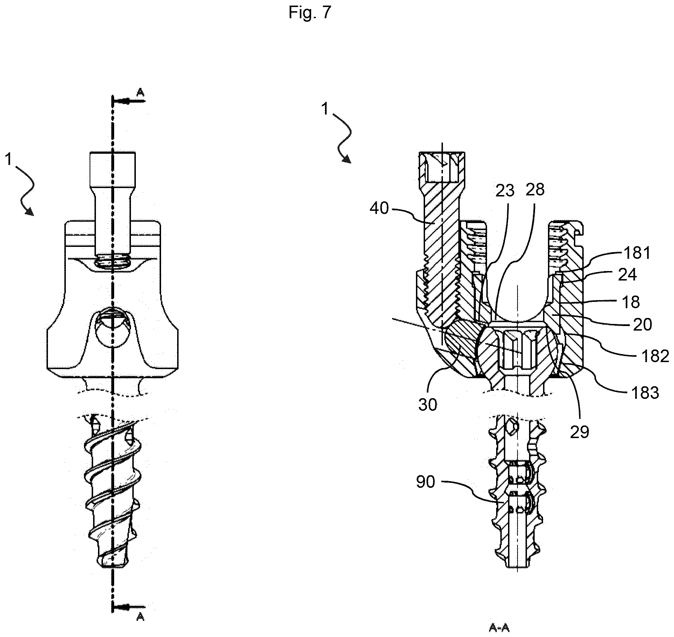

Damit das Druckstück innerhalb des Gabelkopfs 10 optimal geklemmt werden kann, ist im Kugelaufnahmebereich 19 wenigstens ein Innen-Konus-Abschnitt 183 definiert, welcher kongruent zum Konus 271 des Druckstücks 20 vorgesehen ist und bei Betätigung des Verriegelung-Elements 60 zur winkelstabilen Klemmung des Knochenanker-Kopfbereichs 91 mit dem Gabelkopf 10 führt (Fig. 6a,b und Fig. 7). Auch hier ist es erforderlich, dass das Druckstück eine seitliche Öffnung oder einen wenigstens teilweisen Ausschnitt 23 besitzt durch den das Fixier-Element 30 geführt ist und darin sich bewegen kann. Dadurch wird erreicht, dass das Druckstück 20 bei aktivierter temporärer Klemmung nicht belastet wird.

In Fig. 7 ist zu entnehmen, dass die Gabelkopf-Schenkel 11, 12 jeweils eine in proximaler Richtung 101 wirksame Abstützflache 181 mit einer Hinterschneidung bereitstellen, und das Druckstück 20 am proximalen Ende nach radial außen gerichtete Vorsprünge 24 besitzt, wobei die Vorsprünge 24 nach radial innen federelastisch gestaltet sind, so dass das Druckstück 20 aus proximaler Richtung kommend 101 in den Gabelkopf 10 gesteckt werden kann. Dabei verrasten die Vorsprünge des Druckstücks 24 mit den Abstützflachen 181 und das Druckstück 20 wird in proximaler Richtung 101 gesichert aber nicht kraftbeaufschlagt. In Fig. 7 ist auch zu erkennen, dass der Gabelkopf 10 in der Durchgangsöffnung 18 einen Bereich besitzt, welcher wenigstens abschnittsweise einen größeren Innendurchmesser 182 besitzt als der Kerndurchmesser des Gewindes 16. Dadurch ist es möglich, wenn das Druckstück noch nicht vollständig mit dem Gabelkopf 10 in der finalen Position verrastet ist, dass dem federelastischen Konus-Bereich 27 des Druckstücks im Gabelkopf 10 auf Höhe der Innendurchmesser-Erweiterung 182 entsprechender Platz zum Aufspreizen für die Montage mit dem Knochenanker-Kopfbereich 91 bereitgestellt wird.

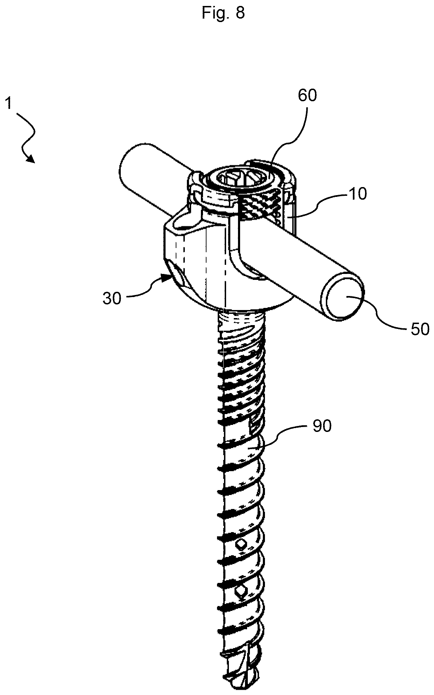

In Fig. 8 dargestellt ist, wenn das Stell-Element 40 Teil der Osteosynthesevorrichtung 1 ist, und eine Sollbruchstelle 45 besitzt, dass nur ein Teil des Stell-Elements 40 nach dem finalen Verriegeln mit dem Verbindungsstab 50 und dem Verriegelungs-Element 60 im Patienten verbleibt. Ein gleiches Bild ergibt sich, wenn das Stell-Element 40 Teil eines Instruments ist und nach dem finalen Verriegeln mit dem Verbindungsstab 50 und dem Verriegelungs-Element 60 aus dem Patienten entfernt ist.

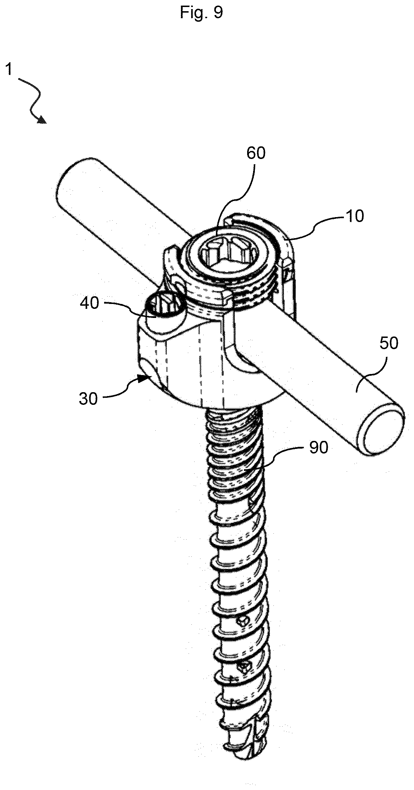

Alternativ kann das Stell-Element 40 auch komplett als Teil der Osteosynthesevorrichtung 1 vorgesehen sein. Nach dem finalen Verriegeln mit dem Verbindungsstab 50 und dem Verriegelungs-Element 60 verbleibt es im Patienten (Fig. 9. Vorteilhaft ist es dann, wenn das Stell-Element 40 nicht über das proximale Ende 101 des Gabelkopfes 10 hinausragt.

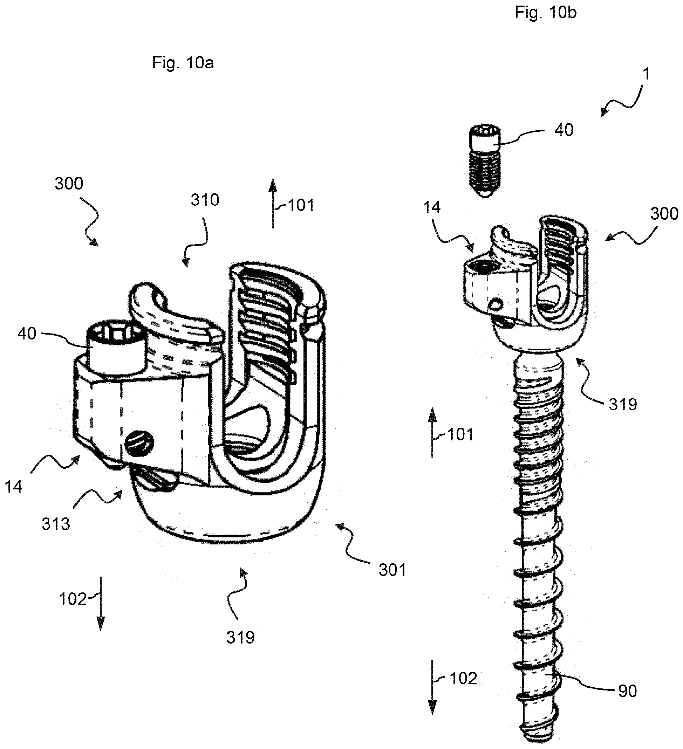

Fig. 10a zeigt ein Befestigungssystem 300 in einer isometrischen Ansicht. Das proximale Ende des Befestigungssystems 300 ist entsprechend einer proximalen Richtung 101 nach oben hin ausgerichtet und dessen distales Ende entsprechend einer distalen Richtung 102 nach unten hin. Das Befestigungssystem 300 weist einen Hauptkörper 301 auf. Proximal weist der Hauptkörper 301 einen ersten Aufnahmebereich 310 auf. Distal weist der Hauptkörper 301 einen zweiten Aufnahmebereich 319 auf. Seitlich distal weist der Hauptkörper 301 einen dritten Aufnahmebereich 313 auf. Seitlich proximal weist der Hauptkörper 301 eine Axial-Öffnung 14 auf. In die Axial-Öffnung 14 ist ein Stell-Element 40 aufgenommen.

Fig. 10b zeigt eine Osteosynthesevorrichtung 1 mit dem in Fig. 10a dargestellten Befestigungssystem 300 in einer isometrischen Ansicht. Das Stell-Element 40 ist entlang der proximalen Richtung 101 von der Axial-Öffnung 14 beabstandet dargestellt, sodass der proximale Eingang der Axial-Öffnung 14 sichtbar ist. In den zweiten Aufnahmebereich 319 ist ein Knochenanker 90 in Neutralstellung aufgenommen. Im Vergleich der Fig. 10a und 10b ist erkennbar, dass das Befestigungssystem 300 modular vorgesehen ist, das heißt der Knochenanker 90 ist modular einsteckbar beziehungsweise einklickbar (Fig. 10b) und wieder entfernbar (Fig. 10a).

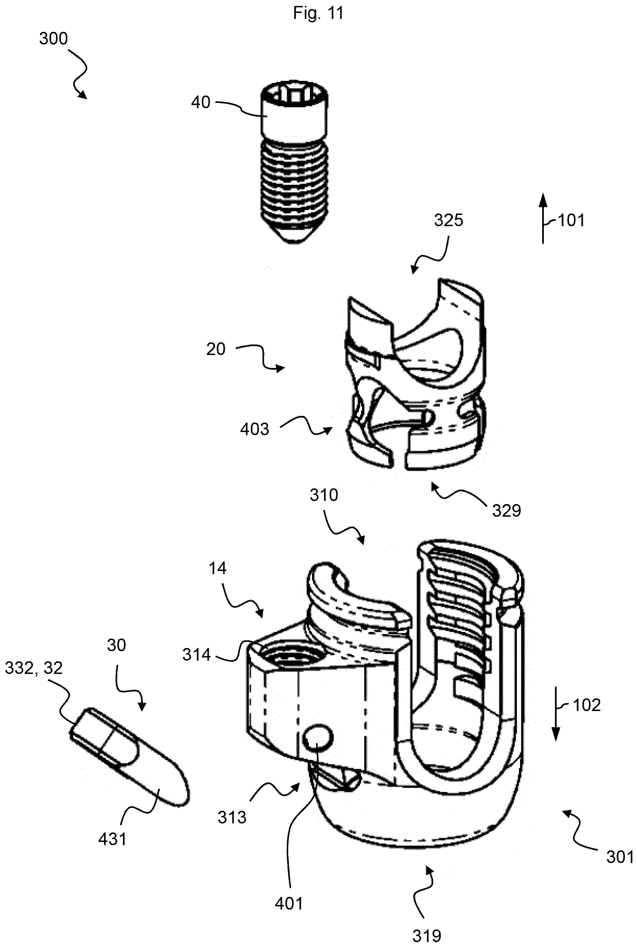

Fig. 11 zeigt das in den Fig. 10a und 10b dargestellte Befestigungssystem 300 in einer Explosionsansicht. Das Befestigungssystem 300 weist ein Druckstück 20 auf, das in distaler Richtung 101 über dem ersten Aufnahmebereich 310 des Hauptkörpers 301 dargestellt ist. Das Druckstück 20 weist in proximaler Richtung 101 einen ersten Aufnahmebereich 325 auf, der zum ersten Aufnahmebereich 310 des Hauptkörpers 301 äquivalent ausgebildet ist. Das Druckstück 20 weist ferner in distaler Richtung 102 einen zweiten Aufnahmebereich 329 auf, der zum ersten Aufnahmebereich 310 des Hauptkörpers 301 äquivalent ausgebildet ist. Außerdem weist das Druckstück 20 seitlich einen dritten Aufnahmebereich 403 auf, der zum dritten Aufnahmebereich 313 des Hauptkörpers 301 äquivalent ausgebildet ist. Die Axial-Öffnung 14 weist ein Innengewinde 314 auf, dessen proximaler Bereich hier ersichtlich ist. Die Axial-Öffnung 14 weist ferner eine Verdrücköffnung 401 auf, die am distalen Ende des Innengewindes 314 positioniert ist - hier ist lediglich ersichtlich, dass die Verdrücköffnung 401 am distalen Ende der Axial-Öffnung 14 positioniert ist und das in der Axial-Öffnung 14 auf der distal-proximalen Höhe das Innengewinde 314 nicht mehr vorliegt. Das Befestigungssystem 300 weist ferner ein Fixier-Element 30 auf. Das Fixier-Element 30 weist seitlich beziehungsweise proximal einen Lastaufnahmebereich 332 beziehungsweise Kontakt-Bereich 32 auf. Das Fixier-Element 30 weist ferner einen ersten Fixierstift 431 auf.

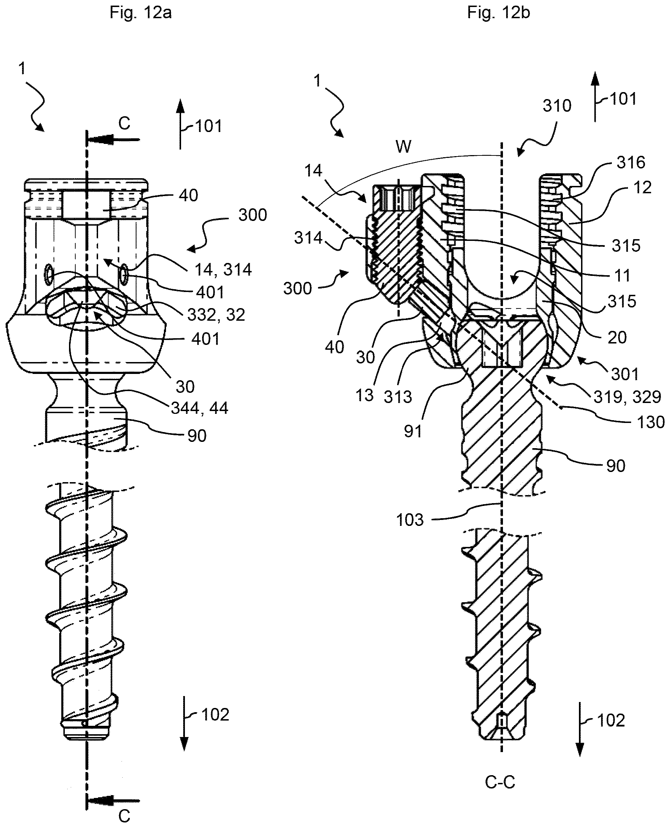

Fig. 12a zeigt die in Fig. 10b dargestellte Osteosynthesevorrichtung 1 in einer Seitenansicht mit einer Schnittebene. Die Schnittebene verläuft zentral durch die Osteosynthesevorrichtung 1 sowie zentral durch das Stell-Element 40 und den Knochenanker 90. Die Axial-Öffnung 14 weist ferner eine zweite Verdrücköffnung 401 auf. Beide Verdrücköffnungen 401 sind jeweils seitlich an der Axial-Öffnung 14 positioniert. Durch diese beiden Verdrücköffnungen 401 kann der distale Bereich des Innengewindes 314 der Axial-Öffnung 14, beispielsweise dessen letzter Gewindegang, verdrückt werden. Das Stell-Element 40 weist distal (siehe distale Richtung 102) einen Lasteinleitungsbereich 344 beziehungsweise Kontakt-Bereich 44 auf. Der Lasteinleitungsbereich 344 ist im Kontakt beziehungsweise Eingriff mit dem Lastaufnahmebereich 332 des Fixier-Elements 30. Die Axial-Öffnung 14 ist entlang der distalen Richtung 102 derart begrenzt beziehungsweise das distale Ende ist derart positioniert, dass von außen her der zweite Aufnahmebereich 319 des Hauptkörpers 301 zugänglich ist. Wird das Stell-Element 40 in die Axial-Öffnung 14 soweit eingeschraubt, dass das proximale Ende des Stell-Elements 40 in die Nähe des proximalen Endes der Axial-Öffnung 14 gelangt, steht das distale Ende des Stell-Elements 40 aus der Axial-Öffnung 14 heraus, ist also weiter entlang der distalen Richtung 102 positioniert, als das distale Ende der Axial-Öffnung (vergleiche auch Fig. 12b). Derart fungiert das Stell-Element 40 über dessen distales Ende als Verliersicherung für das Fixier-Element 30.

Fig. 12b zeigt die in den Fig. 10b und 12a dargestellte Osteosynthesevorrichtung 1 in einer Schnittansicht C-C entlang der ebenfalls in Fig. 12a dargestellten Schnittebene. Die Schnittebene verläuft zentral durch die Osteosynthesevorrichtung 1 sowie zentral durch das Stell-Element 40 und den Knochenanker 90. In den ersten Aufnahmebereich 310 ist das Druckstück 20 derart aufgenommen, dass der erste Aufnahmebereich 310 des Hauptkörpers 301 und der erste Aufnahmebereich 325 des Druckstücks 20 sowie der zweite Aufnahmebereich 319 des Hauptkörpers 301 und der erste Aufnahmebereich 329 des Druckstücks 20 einander ergänzen. Entlang der proximalen Richtung 101 weist der Hauptkörper 301 einen ersten Gabelschenkel 11 auf, der einen Gewindeabschnitt 315 aufweist, und einen zweiten Gabelschenkel 12 auf, der einen Gewindeabschnitt 316 aufweist. Beide Gewindeabschnitte 315 und 316 sind zueinander komplementär ausgebildet. Es ist ferner eine Zentralachse 103 dargestellt, die zentral durch den Hauptkörper 301 beziehungsweise dessen ersten Aufnahmebereich 310 und den zweiten Aufnahmebereich 319 des Hauptkörpers 301 sowie zentral durch den zweiten Aufnahmebereich 329 des Druckstücks 20 und den in Neutralstellung positionierten Knochenanker 90 verläuft. Es ist außerdem eine Fixier-Element-Achse 130 dargestellt, die längs entlang des dritten Aufnahmebereichs 313 und dem in diesen aufgenommenen Fixier-Element 30 verläuft. Der Knochenanker 90 weist nach proximal hin (vergleiche proximale Richtung 101) einen Kopfbereich 91 auf. Der Knochenanker 90 beziehungsweise dessen Kopfbereich 91 ist in den zweiten Aufnahmebereich 329 des Druckstücks und in den zweiten Aufnahmebereich 319 des Hauptkörpers 301 in das Befestigungssystem 300 aufgenommen. Der dritte Aufnahmebereich 313 weist eine Quer-Öffnung 13 auf, in der das Fixier-Element 30 aufgenommen beziehungsweise gelagert ist. Der dritte Aufnahmebereich 313 ist in Bezug auf den ersten Aufnahmebereich 310 und den zweiten Aufnahmebereich 319, das heißt abweichend von der sich von proximal nach distal ergebenden Achse versetzt angeordnet. Der Versatz ist dabei anhand der Rotation der Fixier-Element-Achse 130 um einen Punkt im Kopfbereich 91 beziehungsweise im zweiten Aufnahmebereich 319 des Hauptkörpers 301 in der Schnittebene C-C dargestellt. Dabei spannen die Zentralachse 103 und die Fixier-Element-Achse 130 einen Winkel W auf. Der Winkel W kann - sofern das Stell-Element 40 von proximal auf das Fixier-Element 30 auftrifft - theoretisch im Bereich von 0° bis 90° liegen, wobei er vorzugsweise im Bereich von 20° bis 80° liegt. je kleiner der Winkel W ist, desto geringer ist der Platzbedarf insbesondere für die Axial-Öffnung 14 und den dritten Aufnahmebereich 313 des Hauptkörpers 301. Je größer der Winkel W ist, desto größer ist die Klemmwirkung, die mit dem Fixier-Element 30 auf den Knochenanker 90 beziehungsweise dessen Kopfbereich 91 erzeugt werden kann.

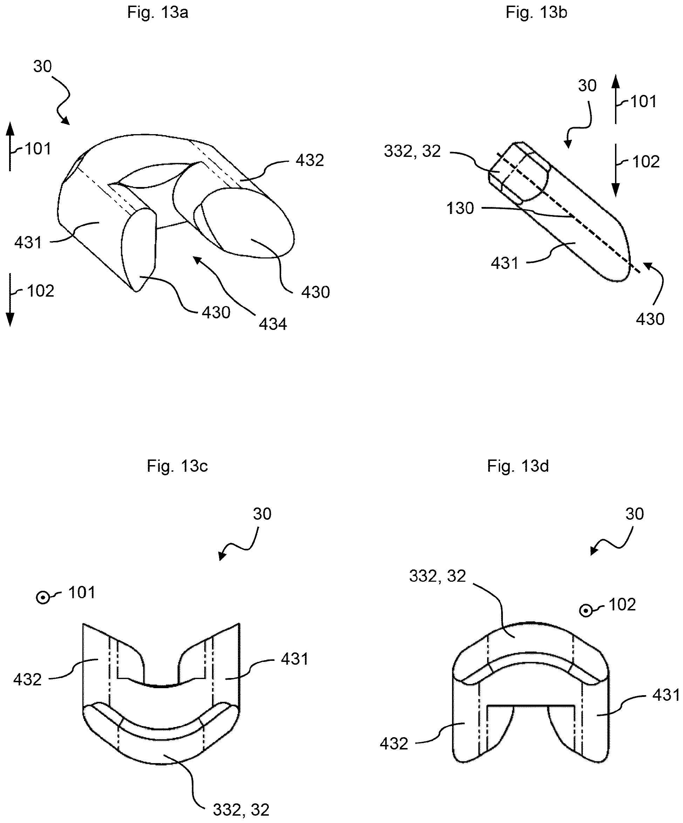

Fig. 13a zeigt ein Fixier-Element 30 mit einem ersten Fixierstift 431 und einem zweiten Fixierstift 432 in einer isometrischen Ansicht. Der erste Fixierstift 431 und der zweite Fixierstift 432 sind nach außen hin jeweils abgerundet ausgebildet. Der erste Fixierstift 431 und der zweite Fixierstift 432 weisen an ihrem distalen Ende jeweils eine Kontaktfläche 430 auf. Zwischen dem ersten Fixierstift 431 und dem zweiten Fixierstift 432 befindet sich ein Freiraum 434.

Fig. 13b zeigt das in Fig. 13a dargestellte Fixier-Element 30 in einer Seitenansicht. Das Fixier-Element 30 ist dabei distal-proximal so wie in den Fig. 12a und 12b dargestellt ausgerichtet. Die Kontaktflächen 430 sind dabei abgeschrägt ausgeführt, sodass trotz des mit dem Winkel W versetzt angeordneten Fixier-Elementes 30 (vergleiche Fig. 12b) diese Kontaktflächen 430 über eine großen Bereich den Knochenanker 90 beziehungsweise dessen Kopfbereich 91 berühren. Die Kontaktflächen 430 sind darüber hinaus nach innen hin, das heißt in Richtung des Freiraums 434 hin, abgeschrägt ausgebildet. Durch beide vorgenannten Abschrägungen, das heißt nach innen hin und in proximaler Richtung 101 beziehungsweise distaler Richtung 102 werden sphärisch-konkav gekrümmte Kontaktflächen 430 erzeugt, die sich unabhängig von der Verdrehposition des Knochenankers 90 über einen großen Bereich an dessen Kopfbereich 91 anschmiegen. Für eine andersartige Geometrie des Kopfbereiches 91 können die Kontaktflächen 430 entsprechend andersartig gekrümmt ausgebildet sein. Der links dargestellte Lastaufnahmebereich 332 beziehungsweise Kontakt-Bereich 32 ist im Wesentlichen senkrecht zur Hauptrichtung des Fixier-Elementes 30, das heißt senkrecht zur Fixier-Element-Achse 130 ausgebildet beziehungsweise orientiert. Dasselbe gilt für den Lasteinleitungsbereich 344 beziehungsweise Kontakt-Bereich 44 des Stell-Elementes 40 - auch dieser ist senkrecht zur Fixier-Element-Achse 130 ausgebildet (siehe Fig. 12b). Dementsprechend sind der Lastaufnahmebereich 332 und der Lasteinleitungsbereich 344 im Wesentlichen parallel zueinander positioniert, sodass ein möglichst großer Lastübertragungsbereich und damit eine möglichst sichere Lastübertragung resultiert. Die Kontaktflächen 430 können mit einer erhöhten Oberflächenrauigkeit ausgebildet sein, so wie dies beispielsweise bei dem Kontaktbereich 31 des in den Fig. 5a und 5b dargestellten Fixier-Elementes 30 ausgeführt ist.

Fig. 13c zeigt das in den Fig. 13a und 13b dargestellte Fixier-Element 30 in einer Aufsicht. Fig. 13d zeigt das in den Fig. 13a bis 13c dargestellte Fixier-Element 30 von unten. Das Fixier-Element 30 ist in beiden Fig. 13c und 13d distal-proximal so wie in den Fig. 12a, 12b, 13a und 13b dargestellt ausgerichtet.

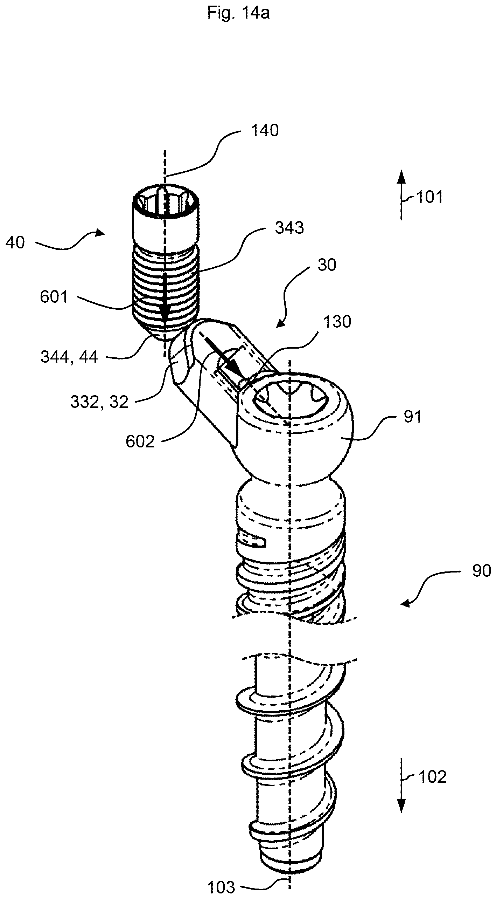

Fig. 14a zeigt ein Stell-Element 40 in Eingriff mit dem in den Fig. 13a bis 13d dargestellten Fixier-Element 30 und einem Knochenanker 90 in einer isometrischen Ansicht. Das Fixier-Element 30 weist ein Außengewinde 343 auf, das komplementär zu dem Innengewinde 314 der Axial-Öffnung 14 ausgebildet ist (vergleiche beispielsweise Fig. 12b). Das Stell-Element 40 ist in der hier nicht dargestellten Axial-Öffnung 14 derart eingeschraubt, dass sich der Lasteinleitungsbereich 344 beziehungsweise Kontakt-Bereich 44 im Eingriff mit dem Lastaufnahmebereich 332 beziehungsweise Kontakt-Bereich 32 befindet (vergleiche beispielsweise Fig. 12b). Dabei resultiert entlang einer Stell-Element-Achse 140 in Bezug auf das Stell-Element 40 eine Wirkrichtung 601. Diese Wirkrichtung 601 verläuft parallel zu der Zentralachse 103, da diese wiederum parallel zu der Axial-Öffnung 14 verläuft (vergleiche Fig. 12b), und gleichgerichtet zur distalen Richtung 102. Zwischen dem Lasteinleitungsbereich 344 und dem Lastaufnahmebereich 332 wird vom Stell-Element 40 die Last auf das Fixier-Element 30 übertragen, wobei der Ausrichtung des Fixier-Elementes 30, das heißt der Fixier-Element-Achse 130 folgend in Bezug auf das Fixier-Element 30 die Wirkrichtung 602 resultiert. Dieser Wirkrichtung 602 entsprechend werden die Kontaktflächen 430 auf den Knochenanker 90 beziehungsweise dessen Kopfbereich 91 gepresst (vergleiche Fig. 13a).

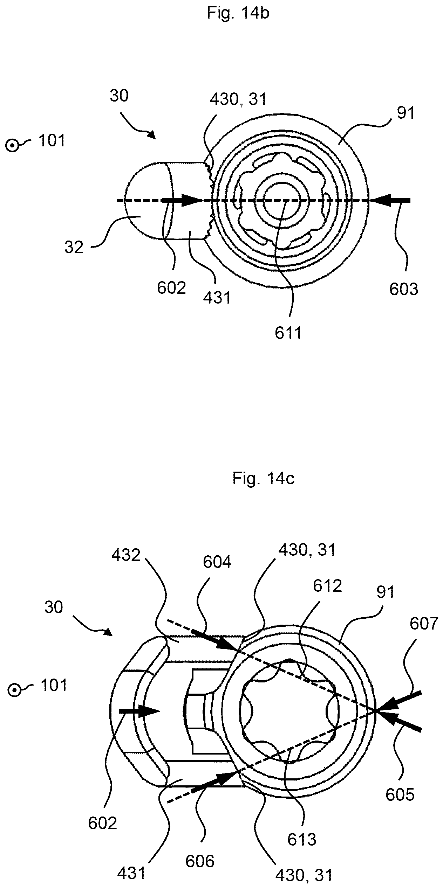

Fig. 14b zeigt ein Fixier-Element 30 mit einem ersten Fixierstift 431 im Eingriff mit einem Kopfbereich 91 eines Knochenankers 90 in einer Aufsicht. Das Fixier-Element 30 ist dabei derart auf den Kopfbereich 91 gepresst, dass dessen erster Fixierstift 431 beziehungsweise die Kontaktfläche 430/der Kontakt-Bereich 31 berührend auf den Kopfbereich 91 gepresst wird, wodurch eine Wirkrichtung 602 resultiert, die im Wesentlichen senkrecht zur Kontaktfläche 430 beziehungsweise zum Kontakt-Bereich 31 verläuft. Dadurch wird der Kopfbereich 91 in den zweiten Aufnahmebereich 319 des Hauptkörpers 301 beziehungsweise in den zweiten Aufnahmebereich 329 des Druckstücks 20 gepresst (beide hier nicht dargestellt, vergleiche aber Fig. 3 und 12b), wodurch eine der Wirkrichtung 602 entgegen gerichtete Wirkrichtung 603 resultiert. Die einander entgegengesetzten Wirkrichtungen 602 und 603 verlaufen auf einer Wirkachse 611, die zentral durch den Kopfbereich 91 verläuft. Derart wird über eine Zwei-Punkt-Klemmung eine sichere Klemmwirkung erzeugt.

Fig. 14c zeigt das in den Fig. 13a bis 13d dargestellte Fixier-Element 30 in der in Fig. 14a dargestellten Eingriffsstellung mit dem Kopfbereich 91 des Knochenankers 90 in einer Aufsicht. Entlang der Wirkrichtung 602 (die wie in Fig. 14b dargestellt verläuft) wird das Fixier-Element 30 auf den Kopfbereich 91 gepresst. An den dadurch resultierenden Berührungsflächen zwischen der Kontaktfläche 430/dem Kontakt-Bereich 31 des ersten Fixierstiftes 431 und der Kontaktfläche 430/dem Kontakt-Bereich 31 des zweiten Fixierstiftes 432 resultieren wiederum in Bezug auf den ersten Fixierstift 431 die Wirkrichtung 606 und in Bezug auf den zweiten Fixierstift 432 die Wirkrichtung 604. Diese beiden Wirkrichtungen 604, 606 sind dabei im Wesentlichen senkrecht zur/zum jeweiligen Kontaktfläche 430/Kontakt-Bereich 31 orientiert. Dadurch wird der Kopfbereich 91 in den zweiten Aufnahmebereich 319 des Hauptkörpers 301 beziehungsweise in den zweiten Aufnahmebereich 329 des Druckstücks 20 gepresst (beide hier nicht dargestellt, vergleiche aber 12b), wodurch jeweils eine den Wirkrichtungen 604, 606 entgegen gerichtete Wirkrichtung 605 (entgegengesetzt zur Wirkrichtung 604), 607 (entgegengesetzt zur Wirkrichtung 606) resultiert. Diese beiden Wirkrichtungen 605, 607 resultieren ferner auf der den Ansatzpunkten der Wirkrichtungen 604, 606 entgegengesetzten Seite des Kopfbereiches 91. Die einander entgegengesetzten Wirkrichtungen 604 und 605 sowie 606 und 607 verlaufen jeweils auf einer Wirkachse 612 (entsprechend den Wirkrichtungen 604 und 605) und 613 (Wirkrichtungen 606 und 607), die jeweils dezentral durch den Kopfbereich 91 verlaufen. Die beiden Wirkrichtungen 605 und 607 setzen im selben Bereich des Kopfbereiches 91 an. Derart wird über eine Drei-Punkt-Klemmung eine sichere Klemmwirkung erzeugt, die im Vergleich zu der in Fig. 14b dargestellten Klemmwirkung nochmal verstärkt ist.

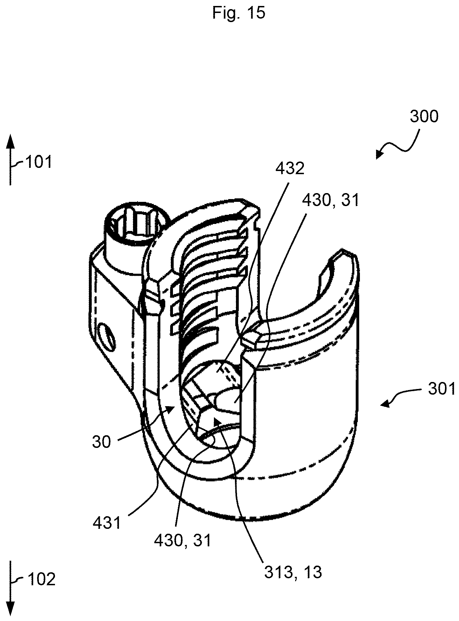

Fig. 15 zeigt das in Fig. 10a dargestellte Befestigungssystem 300 in einer anderen isometrischen Ansicht. In dieser Darstellung ist kein Druckstück 20 dargestellt. Ferner ist das distale Ende des dritten Aufnahmebereiches 313 des Hauptkörpers 301 beziehungsweise das distale Ende der Quer-Öffnung 13 dargestellt, aus dem das distale Ende des Fixier-Elementes 30 herausragt beziehungsweise dessen Kontaktflächen 430/Kontakt-Bereiche 31 herausragen. Der Außenkontur des Fixier-Elements 30 (vergleiche Fig. 13a) folgend ist die Innenkontur der Quer-Öffnung 13 im Querschnitt näherungsweise als Langloch ausgebildet. Um eine sichere Führung des Fixier-Elements 30 zu ermöglichen ist dieser Querschnitt der Quer-Öffnung 13 dabei von proximal nach distal konstant (vergleiche auch Fig. 12a).

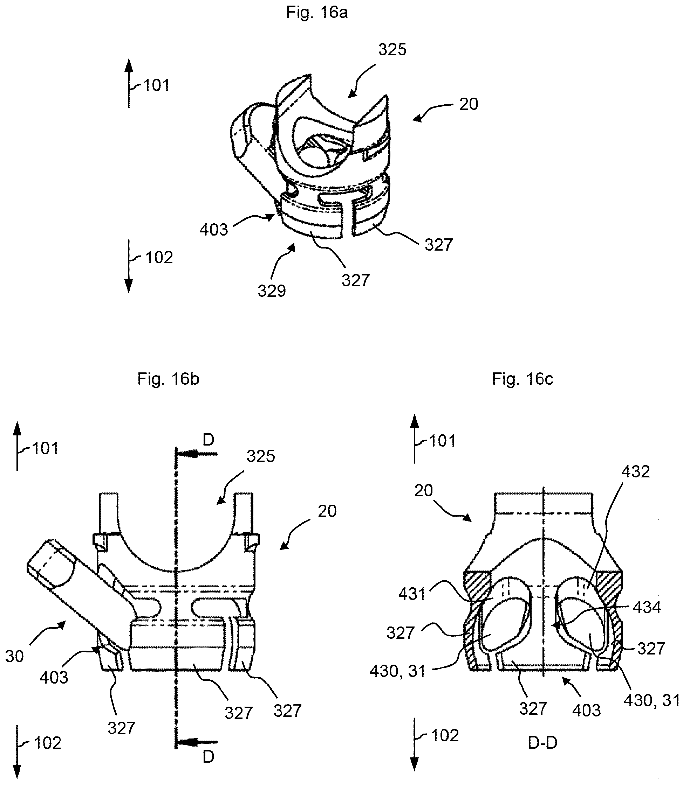

Fig. 16a zeigt das in den Fig. 13a bis 13d dargestellte Fixier-Element 30 im Eingriff mit einem Druckstück 20 in einer isometrischen Ansicht. Das Druckstück 20 weist mehrere Federelemente 327 auf, beispielsweise radial verteilt vier Federelemente 327. Der erste Aufnahmebereich 325 des Druckstücks 20 ist dabei in proximale Richtung 101 verjüngend ausgebildet. In dem dritten Aufnahmebereich 403 des Druckstücks 20 ist das Fixierelement 30 in der ebenfalls in den Fig. 12a, 12b, 13a und 13b dargestellten distal-proximalen Ausrichtung aufgenommen.

Fig. 16b zeigt das in Fig. 16a dargestellte Fixier-Element 30 in der ebenfalls in Fig. 16a dargestellten Eingriffstellung mit dem Druckstück 20 in einer Seitenansicht mit einer Schnittebene. Auch hier ist das Fixierelement 30 in dem dritten Aufnahmebereich 403 des Druckstücks 20 in der ebenfalls in den Fig. 12a, 12b, 13a, 13b und 16a dargestellten distal-proximalen Ausrichtung aufgenommen. Erkennbar sind hier drei Federelemente 327, wobei das Druckstück 20 ferner ein viertes Federelement 327 aufweist, das durch das mittig dargestellte Federelement 327 verdeckt wird (siehe Fig. 16c). Das links dargestellte Federelement 327 befindet sich im dritten Aufnahmebereich 403.

Fig. 16c zeigt das in den Fig. 16a und 16b dargestellte Fixier-Element 30 in der ebenfalls in den Fig. 16a und 16b dargestellten Eingriffstellung mit dem Druckstück 20 in einer Schnittansicht D-D entlang der ebenfalls in Fig. 16b dargestellten Schnittebene. Dabei ist das im dritten Aufnahmebereich 403 angeordnete Federelement 327 derart ausgebildet, dass der erste Fixierstift 431 und der zweite Fixierstift 432 an diesem vorbeigleiten können, ohne dass dieses Federelement 327 dabei in kraftschlüssige Verbindung mit dem ersten Fixierstift 431 und/oder zweiten Fixierstift 432 tritt. In distale Richtung 102 verdickt sich das im dritten Aufnahmebereich 403 angeordnete Federelement 327 unterhalb des ersten Fixierstiftes 431 und des zweiten Fixierstiftes 432. Ebenso verdickt sich das im dritten Aufnahmebereich 403 angeordnete Federelement 327 in proximale Richtung 101 oberhalb des ersten Fixierstiftes 431 und des zweiten Fixierstiftes 432. Das im dritten Aufnahmebereich 403 angeordnete Federelement 327 verdeckt in der in Fig. 16c dargestellten Ansicht den Freiraum 434 beziehungsweise gleitet in diesen ein (vergleiche beispielsweise Fig. 13a). Derart wird auch eine Verliersicherung des Druckstücks 20 durch das Fixier-Element 30 erreicht.

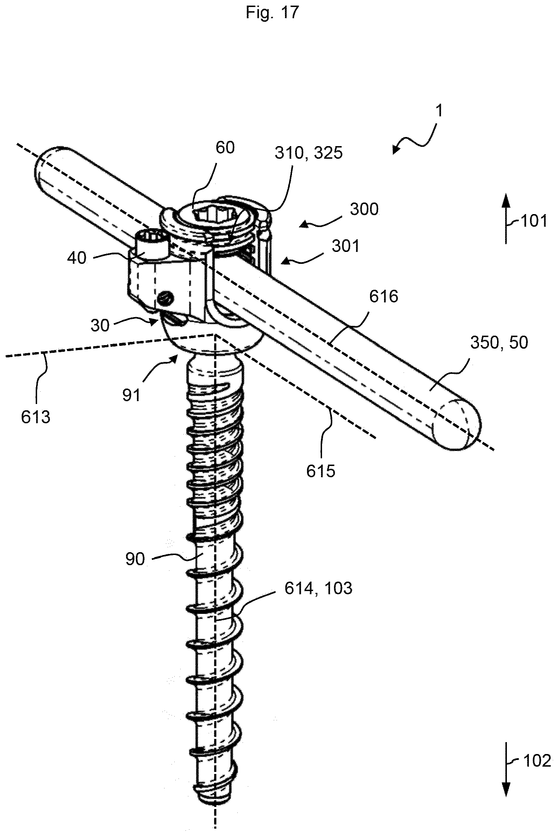

Fig. 17 zeigt die in den Fig. 10b, 12a und 12b dargestellte Osteosynthesevorrichtung 1 mit einem Verbindungselement 350 in einer isometrischen Ansicht. Das Verbindungselement 350 ist hierbei als Verbindungsstab 50 ausgeführt. Der Verbindungsstab 50 ist rotationssymmetrisch ausgebildet. Der Verbindungsstab 50 ist in den ersten Aufnahmebereich 310 des Hauptkörpers 301 sowie in den ersten Aufnahmebereich 325 des Druckstücks 20 aufgenommen und mittels eines Verriegelungs-Elementes 60 gesichert beziehungsweise vorgesichert. Würde das Verriegelungs-Element 60 weiter angezogen werden, würde der Verbindungsstab 50 weiter auf das Druckstück 20 (hier nicht ersichtlich, vergleiche Fig. 12b) gepresst und letztlich eine finale Fixierung des Knochenankers 90 erreicht werden. Ferner würde dadurch eine finale Fixierung des Verbindungsstabes 50 in Bezug auf das Befestigungssystem 300 resultieren, sodass der Verbindungsstab 50 weder in die distale Richtung 101 aus dem Befestigungssystem 300 entfernbar ist, noch entlang der Längsachse des Verbindungsstabes 50 seitlich aus dem Befestigungssystem 300 entfernbar ist. Insbesondere in Bezug zum Befestigungssystem 300 seitliche Fixierung wird hier durch eine Wirkachse 616 verdeutlicht, die parallel zur beziehungsweise entlang der Längsachse des Verbindungsstabes 50 verläuft. Hier dargestellt ist jedoch der Zustand des Vorfixierens, wie er durch Anziehen des Stell-Elements 40 und dadurch Pressen des Fixier-Elements 30 auf den Kopfbereich 91 (hier nicht ersichtlich, vergleiche Fig. 12b) erreicht wird und sich aus der Zusammenschau der Fig. 14a und 14c ergibt. Mit der in Fig. 14c dargestellten Drei-Punkt-Klemmung ergeben sich in Bezug auf den Kopfbereich 91 beziehungsweise Knochenanker 90 drei zueinander senkrechte Wirkachsen 613, 614, 615, die einen Klemmwirkraum aufspannen, innerhalb dessen der Knochenanker 90 in Bezug auf seine Ausrichtung und Lage gegenüber dem Befestigungssystem 300 vorfixiert wird beziehungsweise ist. Die drei Wirkachsen 613, 614, 615 schneiden sich dabei im Kopfbereich 91. Die Wirkachse 614 verläuft dabei zumindest teilweise deckungsgleich zur Zentralachse 103. Die Wirkachse 615 verläuft parallel zu der Wirkachse 616. Die Wirkachse 613 verläuft senkrecht zu den Wirkachsen 614 und 615 und durch den Kopfbereich 91. Die durch die in Fig. 17 dargestellte Vorfixierung erreichte Klemmwirkung wird insbesondere entlang der Längsachse des Verbindungsstabes 50 erreicht, was wie oben beschrieben durch die Wirkachse 615 dargestellt ist. Dabei wird im Unterschied zu der in Fig. 14b dargestellten Zwei-Punkt-Klemmung mit der in den Fig. 14c und 17 dargestellten Drei-Punkt-Klemmung insbesondere die Klemmwirkung entlang und in Richtung der Wirkachse 615 also entlang der Längsachse des Verbindungsstabes 50 verstärkt.

### Bezugszeichenliste

- 1: Osteosynthesevorrichtung
- 10: Gabelkopf
- 11: (erster) Gabelschenkel
- 12: (zweiter) Gabelschenkel
- 13: Quer-Öffnung
- 14: Axial-Öffnung
- 16: Gewinde
- 17: Vorsprünge, Öffnungen, Nuten, Stege, Profile oder sonstige Merkmale
- 18: Durchgangsöffnung
- 19: Kugelkopfaufnahmebereich
- 20: Druckstück
- 21: Schenkel (des Druckstücks)
- 22: Schenkel (des Druckstücks)
- 23: Ausschnitt
- 24: Vorsprung
- 25: Stablager
- 26: Schlitz
- 27: Arm
- 28: Durchgangsöffnung
- 29: Knochenanker-Kopfbereich
- 30: Fixier-Element
- 31: Kontakt-Bereich
- 32: Kontakt-Bereich
- 40: Stell-Element
- 42: Werkzeugansatzstelle
- 43: Gewinde (des Stell-Elements)
- 44: Kontakt-Bereich
- 45: Sollbruchstelle
- 50: Verbindungsstab
- 60: Verriegelungs-Element
- 90: Knochenanker
- 91: Kopfbereich (des Knochenankers)
- 92: Werkzeugansatzstelle (des Knochenankers)
- 93: Knochengewinde
- 101: proximale Richtung
- 102: distale Richtung
- 103: Zentralachse
- 130: Fixier-Element-Achse
- 140: Stell-Element-Achse
- 181: Abstützflache
- 182: Innendurchmesser
- 183: Innen-Konus-Abschnitt
- 271: Konus
- 300: Befestigungssystem
- 301: Hauptkörper
- 310: erster Aufnahmebereich (des Hauptkörpers)
- 313: dritter Aufnahmebereich (des Hauptkörpers)
- 314: Innengewinde (der Axial-Öffnung)
- 315: Gewindeabschnitt (des ersten Gabelschenkels)
- 316: Gewindeabschnitt (des zweiten Gabelschenkels)
- 319: zweiter Aufnahmebereich (des Hauptkörpers)
- 323: Quer-Öffnung (des Druckstücks)
- 325: erster Aufnahmebereich (des Druckstücks)
- 327: Federelement
- 329: zweiter Aufnahmebereich (des Druckstücks)
- 332: Lastaufnahmebereich
- 343: Außengewinde (des Stell-Elementes)
- 344: Lasteinleitungsbereich
- 350: Verbindungselement
- 352: Längsachse (des Verbindungselementes)
- 401: Verdrücköffnung
- 403: dritter Aufnahmebereich (des Druckstücks)
- 430: Kontaktfläche (des Fixierstifts)
- 431: erster Fixierstift
- 432: zweiter Fixierstift
- 434: Freiraum
- 501: erste Öffnung (der Quer-Öffnung)
- 502: zweite Öffnung (der Quer-Öffnung)
- 601: Wirkrichtung
- 602: Wirkrichtung
- 603: Wirkrichtung
- 604: Wirkrichtung
- 605: Wirkrichtung
- 606: Wirkrichtung
- 607: Wirkrichtung
- 611: Wirkachse
- 612: Wirkachse
- 613: Wirkachse
- 614: Wirkachse
- 615: Wirkachse
- 616: Wirkachse
- S1: Stellung
- S2: Stellung
- W: Winkel

## Patentansprüche

1. Befestigungssystem (300) für eine Osteosynthesevorrichtung (1) zur Wirbelsäulenbehandlung, aufweisend einen Hauptkörper (301) mit
einem ersten Aufnahmebereich (310), der zur Aufnahme eines Verbindungselementes (350) ausgebildet ist und der als u-förmiger Gabelkopf (10) ausgebildet ist, der einen ersten Gabelschenkel (11) und einen zweiten Gabelschenkel (12) aufweist.
einem zweiten Aufnahmebereich (319), der zur Aufnahme eines Knochenankers (90) ausgebildet ist, einem dritten Aufnahmebereich (313) aufweisend eine lineare Quer-Öffnung (13), wobei die Quer-Öffnung (13) an ihrem ersten Ende eine erste Öffnung (501) und an ihrem zweiten Ende eine zweite Öffnung (502) aufweist, und
einer Axial-Öffnung (14) zur Aufnahme und Führung eines Stell-Elementes (40),
ferner aufweisend ein Fixier-Element (30) zum Fixieren des Knochenankers (90) aufweisend einen ersten Fixierstift (431), und einen Lastaufnahmebereich (332), wobei der erste Fixierstift (431) über die erste Öffnung (501) in die Quer-Öffnung (13) einführbar ist und das Fixier-Element (30) länger als die Quer-Öffnung (13) ist, und wobei der erste Fixierstift (431) an seinem dem Lastaufnahmebereich (332) entfernten Ende eine Kontaktfläche (430) aufweist, und
ein Stell-Element (40) aufweisend einen Lasteinleitungsbereich (344), wobei das Stell-Element (40) mittels der Axial-Öffnung (14) derart positionierbar und führbar ist, dass mittels des durch den Lasteinleitungsbereich (344) auf den Lastaufnahmebereich (332) ausgeübten Druckes das Fixier-Element (30) entlang der Quer-Öffnung (13) in Richtung des zweiten Aufnahmebereiches (319) führbar ist.

2. Befestigungssystem (300) nach Anspruch 1, wobei der dritte Aufnahmebereich (313) derart ausgestaltet ist, dass die Quer-Öffnung (13) mit einem Versatz zur Zentralachse (103) des in Neutralstellung in den zweiten Aufnahmebereich (319) aufgenommenen Knochenankers (90) vorgesehen ist, wobei der Versatz mindestens 20°, vorzugsweise 30° und besonders bevorzugt 40° sowie maximal 80°, vorzugsweise 70° und besonders bevorzugt 60° beträgt

3. Befestigungssystem (300) nach einem der vorhergehenden Ansprüche, wobei der Lasteinleitungsbereich (344) und der Lastaufnahmebereich (332) zueinander komplementär ausgebildet sind.

4. Befestigungssystem (300) nach einem der vorhergehenden Ansprüche, wobei das Befestigungssystem (300) ferner ein Druckstück (20) aufweist, wobei das Druckstück (20) einen zu dem ersten Aufnahmebereich (310) des Befestigungssystems (300) äquivalenten ersten Aufnahmebereich (325), einen zu dem zweiten Aufnahmebereich (319) des Befestigungssystems (300) äquivalenten und mehrere Federelemente (327) aufweisenden zweiten Aufnahmebereich (329) sowie einen zu dem dritten Aufnahmebereich (313) des Hauptkörpers (301) äquivalenten und eine lineare Quer-Öffnung (323) aufweisenden dritten Aufnahmebereich (403) aufweist, und wobei das Druckstück (20) über den ersten Aufnahmebereich (310) des Hauptkörpers (301) in den Hauptkörper (301) einsetzbar ist.

5. Befestigungssystem (300) nach einem der vorhergehenden Ansprüche, wobei die Axial-Öffnung (14) ein Innengewinde (314) aufweist und das Stell-Element (40) ein Außengewinde (343) aufweist, und wobei das Innengewinde (314) der Axial-Öffnung (14) komplementär zu dem Außengewinde (343) des Stell-Elementes (40) ausgebildet ist, wobei insbesondere das Innengewinde (314) der Axial-Öffnung (14) im Wesentlichen parallel zu der Zentralachse (103) des in Neutralstellung in den zweiten Aufnahmebereich (319) aufgenommenen Knochenankers (90) verläuft.

6. Befestigungssystem (300) nach Anspruch 5, wobei die Axial-Öffnung (14) zumindest eine Verdrücköffnung (401) aufweist, wobei die Verdrücköffnung (401) am distalen Ende des Innengewindes (314) positioniert ist, und wobei die Verdrücköffnung (401) derart ausgebildet ist, dass über diese ein Verdrücken des Außengewindes (343) des Stell-Elementes (40) ermöglicht wird.

7. Befestigungssystem (300) nach einem der vorhergehenden Ansprüche, wobei das Fixier-Element (30) ferner einen zweiten Fixierstift (432) aufweist.

8. Befestigungssystem (300) nach einem der vorhergehenden Ansprüche, wobei die Kontaktfläche (430) des ersten Fixierstiftes (431) oder gegebenenfalls des ersten und/oder zweiten Fixierstiftes (432) komplementär zu einer entsprechenden Kontaktfläche (430) des Knochenankers (90) ausgebildet ist.

9. Befestigungssystem (300) nach Anspruch 7 und einem der Ansprüche 4, oder 5, 6 oder 8 in Kombination mit Anspruch 4, wobei ein Federelement (327) in der linearen Quer-Öffnung (323) des Druckstückes (20) angeordnet ist, und wobei dieses Federelement (327) insbesondere mittig in der linearen Quer-Öffnung (323) platziert ist.

10. Befestigungssystem (300) nach Anspruch 9, wobei das in der linearen Quer-Öffnung (323) angeordnete Federelement (327) im distalen Bereich verbreiternd ausgebildet ist.

11. Befestigungssystem (300) nach einem der vorhergehenden Ansprüche, wobei die Kontaktfläche (430) des ersten Fixierstiftes (431) oder gegebenenfalls des ersten und/oder zweiten Fixierstiftes (431, 432) eine lokal erhöhte Oberflächenrauigkeit aufweist.

12. Befestigungssystem (300) nach einem der vorhergehenden Ansprüche, wobei der erste Aufnahmebereich (310) des Hauptkörpers (301) einen Gewinde (16) aufweist.

13. Befestigungssystem (300) nach Anspruch 12, wobei der dritte Aufnahmebereich (313) und die Axial-Öffnung (14) an dem ersten Gabelschenkel (11) angeordnet sind.

14. Osteosynthesevorrichtung (1), insbesondere zur Wirbelsäulenbehandlung, aufweisend einen Knochenanker (90) und ein Befestigungssystem (300) nach einem der vorhergehenden Ansprüche.

## Claims

1. Attachment system (300) for an osteosynthesis device (1) for spinal treatment, having a main body (301) comprising
a first receiving region (310) which is designed to receive a connecting element (350) and which is designed as a U-shaped fork head (10) which has a first fork leg (11) and a second fork leg (12)
a second receiving region (319) which is designed to receive a bone anchor (90),
a third receiving region (313) having a linear transverse opening (13), the transverse opening (13) having a first opening (501) at its first end and a second opening (501) at its second end, and
an axial opening (14) for receiving and guiding an adjusting element (40),
further comprising a fixing element (30) for fixing the bone anchor (90) comprising a first fixing pin (431), and a load receiving area (332), wherein the first fixing pin (431) can be inserted into the transverse opening (13) via the first opening (501) and the fixing element (30) is longer than the transverse opening (13), and wherein the first fixing pin (431) has a contact surface (430) at the end remote to the load receiving area (332), and
an adjusting element (40) having a load introduction region (344), wherein the adjusting element (40) can be positioned and guided by means of the axial opening (14) in such a way that, by means of the load introduced through the load introduction region (344) onto the load receiving area (332), the fixing element (30) can be guided along the transverse opening (13) in the direction of the second receiving area (319).

2. Attachment system (300) according to claim 1, wherein the third receiving region (313) is designed in such a way that the transverse opening (13) is provided with an offset with respect to the central axis (103) of the bone anchor (90) received in the second receiving region (319) in the neutral position, wherein the offset is at least 20°, preferably 30° and particularly preferably 40° and at most 80°, preferably 70° and particularly preferably 60°.

3. Attachment system (300) according to one of the preceding claims, wherein the load introduction area (344) and the load receiving area (332) are complementary to each other.

4. Attachment system (300) according to one of the preceding claims, wherein the attachment system (300) further comprising a thrust piece (20), wherein the thrust piece (20) has a first receiving portion (310) equivalent to the first receiving region (325) of the attachment system (300), a second receiving region (329) which is equivalent to the second receiving region (319) of the attachment system (300) and which has a plurality of spring elements (327), and a third receiving region (403) which is equivalent to the third receiving region (313) of the main body (301) and has a linear transverse opening (323), and wherein the thrust piece (20) can be inserted into the main body (301) via the first receiving region (310) of the main body (301).

5. Attachment system (300) according to one of the preceding claims, wherein the axial opening (14) has an internal thread (314) and the adjusting element (40) has an external thread (343), and wherein the internal thread (314) of the axial opening (14) is formed complementary to the external thread (343) of the adjusting element (40), wherein in particular the internal thread (314) of the axial opening (14) extends substantially parallel to the central axis (103) of the bone anchor (90) received in the second receiving region (319) in the neutral position.

6. Attachment system (300) according to claim 5, wherein the axial opening (14) has at least one compression opening (401), wherein the compression opening (401) is located at the distal end of the inner thread (314), and wherein the compression opening (401) is designed such to enable compression of the outer thread (343) of the adjusting element (40) via said compression opening.

7. Attachment system (300) according to one of the preceding claims, wherein the fixing element (30) also has a second fixing pin (432).

8. Attachment system (300) according to one of the preceding claims, wherein the contact surface (430) of the first fixing pin (431) or optionally of the first and/or second fixing pin (432) is formed complementary to a corresponding contact surface (430) of the bone anchor (90).

9. Attachment system (300) according to claim 7 and one of claims 4, or 5, 6 or 8 in combination with claim 4, wherein a spring element (327) is arranged in the linear transverse opening (323) of the pressure piece (20), and wherein this spring element (327) is placed in particular centrally in the linear transverse opening (323).

10. Attachment system (300) according to claim 9, wherein the spring element (327) arranged in the linear transverse opening (323) is designed to widen in the distal region.

11. Attachment system (300) according to one of the preceding claims, wherein the contact surface (430) of the first fixing pin (431) or optionally of the first and/or second fixing pin (431, 432) has a locally increased surface roughness.

12. Attachment system (300) according to one of the preceding claims, wherein the first receiving area (310) of the main body (301) has a thread (16).

13. The attachment system (300) according to claim 12, wherein the third receiving area (313) and the axial opening (14) are arranged on the first fork leg (11).

14. Osteosynthesis device (1), in particular for spinal treatment, comprising a bone anchor (90) and an attachment system (300) according to any one of the preceding claims.

## Revendications

1. Système de fixation (300) pour un dispositif d'ostéosynthèse (1) pour le traitement de la colonne vertébrale, comprenant un corps principal (301)
doté d'une première zone de réception (310), qui est conçue pour recevoir un élément de liaison (350) et qui est conçue comme une chape (10) en forme de U, qui présente une première branche de chape (11) et une seconde branche de chape (12).
une deuxième zone de réception (319) qui est conçue pour recevoir un ancrage osseux (90),
une troisième zone de réception (313) comprenant une ouverture transversale linéaire (13), dans lequel l'ouverture transversale (13) comprend une première ouverture (501) à sa première extrémité et une seconde ouverture (502) à sa seconde extrémité, et
une ouverture axiale (14) pour recevoir et guider un élément de réglage (40), comprenant en outre un élément de fixation (30) pour fixer l'ancrage osseux (90), comprenant une première tige de fixation (431), et une partie de réception de charge (332), dans lequel la première tige de fixation (431) peut être insérée dans l'ouverture transversale (13) via la première ouverture (501), et l'élément de fixation (30) est plus long que l'ouverture transversale (13), et dans lequel la première tige de fixation (431) présente une surface de contact (430) à son extrémité éloignée de la zone de réception de charge (332), et
un élément de réglage (40) comprenant une zone d'introduction de charge (344), dans lequel l'élément de réglage (40) peut être positionné et guidé au moyen de l'ouverture axiale (14) de telle sorte qu'au moyen de la pression exercée par la zone d'introduction de charge (344) sur la zone de réception de charge (332), l'élément de fixation (30) peut être guidé le long de l'ouverture transversale (13) en direction de la deuxième zone de réception (319).

2. Système de fixation (300) selon la revendication 1, dans lequel la troisième zone de réception (313) est conçue de telle sorte que l'ouverture transversale (13) est prévue avec un décalage par rapport à l'axe central (103) de l'ancrage osseux (90) reçu en position neutre dans la deuxième zone de réception (319), dans lequel le décalage est d'au moins 20°, de préférence 30° et de manière particulièrement préférée 40° ainsi que de 80° au maximum, de préférence 70° et de manière particulièrement préférée 60°.

3. Système de fixation (300) selon l'une des revendications précédentes, dans lequel la zone d'introduction de charge (344) et la zone de réception de charge (332) sont complémentaires l'une de l'autre.

4. Système de fixation (300) selon l'une des revendications précédentes, dans lequel le système de fixation (300) comprend en outre une pièce de pression (20), dans lequel la pièce de pression (20) comprend une première zone de réception (325) équivalente à la première zone de réception (310) du système de fixation (300), une deuxième zone de réception (329) équivalente à la deuxième zone de réception (319) du système de fixation (300) et présentant plusieurs éléments élastiques (327) ainsi qu'une troisième zone de réception (403) équivalente à la troisième zone de réception (313) du corps principal (301) et présentant une ouverture transversale linéaire (323), et dans lequel la pièce de pression (20) peut être insérée dans le corps principal (300) via la première zone de réception (310) du corps principal (301).

5. Système de fixation (300) selon l'une des revendications précédentes, dans lequel l'ouverture axiale (14) présente un filetage intérieur (314) et l'élément de réglage (40) présente un filetage extérieur (343), et dans lequel le filetage intérieur (314) de l'ouverture axiale (14) est complémentaire du filetage extérieur (343) de l'élément de réglage (40), dans lequel, en particulier, le filetage intérieur (314) de l'ouverture axiale (14) s'étend sensiblement parallèlement à l'axe central (103) de l'ancrage osseux (90) reçu en position neutre dans la deuxième zone de réception (319).

6. Système de fixation (300) selon la revendication 5, dans lequel l'ouverture axiale (14) présente au moins une ouverture d'enfoncement (401), dans lequel l'ouverture d'enfoncement (401) est positionnée à l'extrémité distale du filetage intérieur (314), et dans lequel l'ouverture d'enfoncement (401) est réalisée de telle sorte qu'un enfoncement du filetage extérieur (343) de l'élément de réglage (40) est rendu possible par l'intermédiaire de celle-ci.

7. Système de fixation (300) selon l'une des revendications précédentes, dans lequel l'élément de fixation (30) comprend en outre une seconde tige de fixation (432).

8. Système de fixation (300) selon l'une des revendications précédentes, dans lequel la surface de contact (430) de la première tige de fixation (431) ou, le cas échéant, de la première et/ou de la seconde tige de fixation (432) est complémentaire d'une surface de contact correspondante (430) de l'ancrage osseux (90).

9. Système de fixation (300) selon la revendication 7 et l'une des revendications 4, ou 5, 6 ou 8 en combinaison avec la revendication 4, dans lequel un élément élastique (327) est disposé dans l'ouverture linéaire transversale (323) de la pièce de pression (20), et dans lequel cet élément élastique (327) est placé notamment au centre de l'ouverture linéaire transversale (323).

10. Système de fixation (300) selon la revendication 9, dans lequel l'élément élastique (327) disposé dans l'ouverture transversale linéaire (323) est réalisé de manière à s'élargir dans la zone distale.

11. Système de fixation (300) selon l'une des revendications précédentes, dans lequel la surface de contact (430) de la première tige de fixation (431) ou, le cas échéant, de la première et/ou de la seconde tige de fixation (431, 432) présente une rugosité de surface localement accrue.

12. Système de fixation (300) selon l'une quelconque des revendications précédentes, dans lequel la première zone de réception (310) du corps principal (301) comporte un filetage (16).

13. Système de fixation (300) selon la revendication 12, dans lequel la troisième zone de réception (313) et l'ouverture axiale (14) sont disposées sur la première branche de chape (11).

14. Dispositif d'ostéosynthèse (1), notamment pour le traitement de la colonne vertébrale, comprenant un ancrage osseux (90) et un système de fixation (300) selon l'une des revendications précédentes.
